(19) Europäisches Patentamt
European Patent Office
Office européen des brevets

(11) EP 3 457 136 A1

(12) **EUROPEAN PATENT APPLICATION**
published in accordance with Art. 153(4) EPC

(43) Date of publication:
**20.03.2019 Bulletin 2019/12**

(21) Application number: **17796197.6**

(22) Date of filing: **10.05.2017**

(51) Int Cl.:
***G01N 33/543*** *(2006.01)*

(86) International application number:
**PCT/JP2017/017734**

(87) International publication number:
**WO 2017/195838 (16.11.2017 Gazette 2017/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.05.2016 JP 2016096681**

(71) Applicant: **Eiken Kagaku Kabushiki Kaisha**
**Taito-ku**
**Tokyo 110-8408 (JP)**

(72) Inventors:
- **ANDO Sachiko**
  **Shimotsuga-gun**
  **Tochigi 329-0114 (JP)**
- **YOSHINO Manabu**
  **Shimotsuga-gun**
  **Tochigi 329-0114 (JP)**

(74) Representative: **Herzog, Fiesser & Partner**
**Patentanwälte PartG mbB**
**Dudenstrasse 46**
**68167 Mannheim (DE)**

# (54) METHOD FOR CALCULATING RATIO OF MEASUREMENT OBJECT SUBSTANCE TO COMPARISON OBJECT SUBSTANCE, PROGRAM, STORAGE MEDIUM, AND DEVICE

(57) Provided is a method for determining the ratio of a measurement object substance to a comparison object substance using a measuring reagent for the measurement object substance. The method makes it possible to easily determine, from an absorbance measured value, the ratio of the measurement object substance to the comparison object substance, using a single calibration curve (multiplexed calibration curve) which is not dependent on the concentration of the comparison object substance.

Fig. 11B

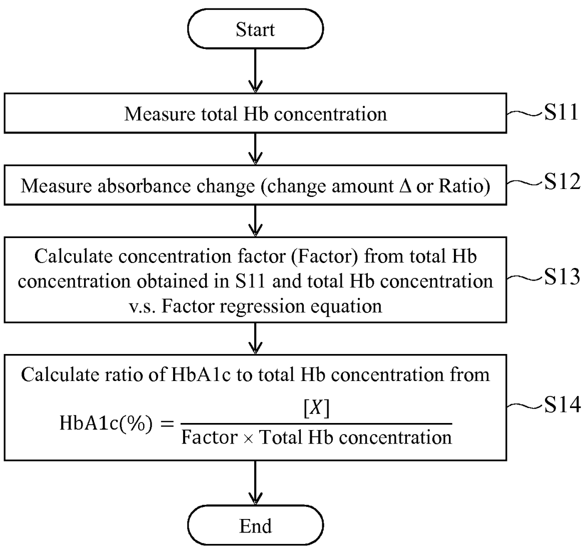


EP 3 457 136 A1

Printed by Jouve, 75001 PARIS (FR)

## Description

Technical Field

The present invention relates to a technique for measuring the ratio of a measurement substance to a comparison object substance.

Background Art

In a specimen examination performed during clinical examination, by measuring chemical substances, such as protein, enzymes, electrolytes, metal ions, lipids, carbohydrates, and vitamins, in a specimen, such as blood, spinal fluid, urine, stool, tissues, or cells collected from the patient, it becomes possible to find abnormality in organs or to learn the state of infection due to a bacterium or a virus. Accordingly, the specimen examination is used as a means for diagnosing a disease, determining a treatment policy, or obtaining an indicator of the effect of a treatment.

In the case of various chemical substances in a specimen as the measurement object, abnormality in an organ of interest is indicated by the amount of a measured value relative to a reference value. Accordingly, the measured value is expressed by concentration, and mg/dL or mol/L is used as the unit, for example. With respect to enzymes and vitamins, activity values are indicated using an enzyme unit or an international unit, such as IU/L.

As a measurement object, blood sugar, i.e., glucose is measured to learn the clinical state (state of high blood sugar value) of a diabetes patient, where mg/dL is used as the unit in Japan, for example. A blood sugar test makes it possible to learn an accurate blood sugar value at the point in time of blood sampling. However, blood sugar values vary constantly (a fasting reference value is less than 110 mg/dL; a reference value two-hours after eating is less than 140 mg/dL), and the range of blood sugar variation (blood sugar state) cannot be learned. That is, the effect of blood sugar control performed as a treatment for the diabetes patient cannot be learned.

In order to learn the effect of blood sugar control, the proportion of glycated proteins which reflects the blood sugar state during a certain period may be used as an indicator. As the indicator, HbA1c or glycoalbumin may be used.

First, HbA1c will be described. Hemoglobin (Hb) is a chromoprotein in red blood cells, and is a heterotetramer consisting of two $\alpha$-subunits and two $\beta$-subunits. The Hb in red blood cells bind to glucose non-enzymatically, i.e., in a blood glucose concentration-dependent manner, as the red blood cells circulate through the body. The Hb bound to glucose is glycated Hb, i.e., HbA1. HbA1 has several types depending on the type of sugar binding to the $\beta$-subunit. HbA1c is where glucose is bound to the N terminal end of the $\beta$-subunit. HbAlc is used as an indicator reflecting the blood sugar state in the past one to two months since the point in time of blood sampling. As a measured value, the ratio, i.e., a relative amount, of HbA1c to the total Hb is used with the unit %.

Glycoalbumin will be described. Albumin occupies a large portion of the protein in blood. Albumin, as it circulates in the body together with blood, binds to sugar, as in the case of hemoglobin, and the albumin bound to sugar is glycated albumin, i.e., glycoalbumin. Glycoalbumin is used as an indicator reflecting the blood sugar state in the past two to three weeks since the point in time of blood sampling. As a measured value, the ratio, i.e., a relative amount, of glycoalbumin to the total albumin is used with the unit %.

Other than HbA1c and glycoalbumin, isozymes (such as lactate dehydrogenase; isozymes that differ in the tissue, cell, or organelle in which they exist, i.e., isozymes having different derivations) are another example in which the ratio (relative amount) of a measurement object substance to a comparison object substance may be used as a measured value.

The reason that the ratio of the measurement object substance to the comparison object substance is used as the measured value is because, for example, the total amount of the comparison object substance may vary depending on the person, sex, age, affecting disease and the like, and therefore the clinical state cannot be accurately learned when denoted by the concentration of the measurement object substance, i.e., by mass per unit volume, for example.

In the case of HbA1c as an example, the total Hb concentration in human blood is in a range of 13.1 to 16.6 g/dL for males and in a range of 12.1 to 14.6 g/dL for females. Thus, there are a male/female difference, an individual difference, and an intraindividual difference in the reference value of total Hb concentration. The total Hb concentration may also greatly vary depending on the presence or absence of a disorder that causes anemic condition (such as iron-deficiency anemia and aplastic anemia), erythrocytosis, or dehydration. Accordingly, even if expressed in terms of HbA1c concentration, as Hb is glycated in a blood glucose concentration-dependent manner, it is impossible to accurately learn the blood sugar state unless the total Hb concentration is looked at, i.e., unless a readout is used that makes it possible to learn the relationship between HbA1c and total Hb concentration simultaneously. In other words, even when the HbA1c concentration is the same, the ratio of HbA1c to the total Hb becomes lower for a person with a high total Hb concentration, and the ratio of HbA1c to the total Hb becomes higher for a person with a low total Hb concentration.

A method for determining, as a measured value, the ratio of a measurement object substance to a comparison object substance will be described with reference to HbA1c as an example. As a first method, a method using HPLC is known. In this method, the ratio of HbA1c to the total Hb is determined directly from the areas of peaks of the measurement object substance and the comparison object substance.

As a second method, an immunoassay (agglutination method or agglutination inhibition method) is known (see Patent Literatures 1, 2, and 3, for example), in which the HbA1c concentration and the total Hb concentration in a sample are respectively determined, and finally the ratio of HbA1c to the total Hb is determined.

Specifically, first, the HbA1c and Hb in the sample are denatured, immunoreaction is caused, and the HbA1c concentration and the total Hb concentration in the sample due to absorbance changes caused by immunoreaction are determined. Then, finally, the ratio of HbA1c to the total Hb is determined. Known examples of the immunoassay include an agglutination method and an agglutination inhibition method.

As a third method, an enzymatic method is known (see Non Patent Literatures 1 and 2), in which the HbA1c concentration and the total Hb concentration in the sample are respectively determined, and then finally the ratio of HbA1c is determined.

Specifically, first, the HbA1c and Hb in the sample are denatured, and the total Hb concentration is determined from absorbance. Then, the HbA1c concentration is determined by measuring absorbance using a color reaction due to a color reagent and hydrogen peroxide, which is generated by causing fructosyl peptide oxidase and further peroxidase to act on glycated dipeptide derived from the N terminal end of a β-subunit produced by protease processing of HbA1c. Finally, the ratio of HbA1c to the total Hb is determined.

Citation List

Patent Literature

Patent Literature 1: JP 2596322 B
Patent Literature 2: JP 3550614 B
Patent Literature 3: JP 5465008 B

Non Patent Literature

Non Patent Literature 1: Clin Chem. 51, A246, 2005
Non Patent Literature 2: Journal of Bioscience and Bioengineering, Vol. 92, No. 2, 62-68. 2014

Summary of Invention

Technical Problem

However, the first method has the problem that the measurement device is large and expensive, and that the column, which is an expendable component, is expensive. In the second and third methods, it is necessary to first determine the HbA1c concentration from the absorbance measured value, using complex computing formulas. This means that it is necessary to maintain a certain ratio (volume ratio) of a reagent and a specimen throughout measurement. Particularly, during an actual measurement for determining the ratio of HbA1c to the total Hb, the step of mixing the reagent and specimen is performed at least twice, i.e., during specimen pretreatment (including hemolyzing processing, diluting processing, or denaturing processing) and during measurement. Accordingly, variations tend to be caused in the volumes of the reagent and specimen, inevitably resulting in a decrease in the reliability of the HbA1c concentration and the total Hb concentration and further of the ratio of HbA1c to the total Hb.

In a reaction system using the immunoassay or the enzymatic method as in the second or third method, an attempt may be made to determine the ratio of HbA1c to the total Hb directly from the absorbance measured value without being affected by the problem of the volume ratio of reagent and specimen. However, as described above, the concentration of the total Hb varies from one specimen to another, and it would be necessary to prepare numerous calibration curves for each total Hb concentration. Thus, the attempt is practically impossible.

In light of the above circumstances, an objective of the present invention is to provide a technique for determining the ratio of a measurement object substance to a comparison object substance, and particularly to provide a technique for determining the ratio of a measurement object substance to a comparison object substance directly from an absorbance measured value when the concentration of the comparison object substance varies depending on the specimen.

Solution to Problem

According to an aspect of the present invention, there is provided a method for determining a ratio of a measurement object substance to a comparison object substance using a measuring reagent for the measurement object substance. In a process for determining, at each concentration of the comparison object substance, a concentration factor such that a plurality of calibration curves, which are obtained by determining an absorbance change amount of each dilution series of the measurement object substance due to the progress of a reaction between the dilution series of the measurement object substance having different concentrations of the comparison object substance and the measuring reagent, converge into a single calibration curve, the method includes:

a step of obtaining the ratio (%) of the measurement object substance to the comparison object substance = [X]/(concentration factor $\times$ concentration of comparison object substance) (1); and
a step of determining a regression equation expressing a relationship between the concentration factor for each concentration of the comparison object substance and the concentration of the comparison object substance.

The step of determining the absorbance change amount may include: a step of determining a change amount in an increase or decrease in absorbance at a characteristic single wavelength due to the progress of a reaction caused by a specimen including the measurement object substance and the measuring reagent; or a step of determining a change amount in an increase or decrease in absorbance at characteristic two wavelengths due to the progress of a reaction caused by the specimen including the measurement object substance and the measuring reagent. The change amount may be a difference or a ratio between a first absorbance at a first wavelength at which the absorbance increases or decreases and a second absorbance at a second wavelength at which the absorbance increases or decreases.
The measuring reagent may be a measuring reagent utilizing any of an immunoassay which includes an agglutination method and an agglutination inhibition method, and an enzymatic method. The method for determining the ratio may include a step of determining the ratio of the measurement object substance to the comparison object substance from: the expression (1); a regression equation of the concentration factor optimized for each concentration of the comparison object substance and the concentration of the comparison object substance; and an absorbance change amount obtained by measuring a specimen including the measurement object substance using the measuring reagent.
The present invention may provide a program for causing a computer to perform the method according to any one of the above.
There may be provided a computer-readable recording medium having the program recorded thereon.
The present invention may also provide a device for determining a ratio of the measurement object substance to the comparison object substance using a measuring reagent for the measurement object substance. The device may include a control unit for performing the method according to any one of the above, and an absorbance measurement unit. Preferably, the control unit and the absorbance measurement unit may be integrally or separately provided.
The present invention may also provide a device including a control unit incorporating the program, or a device including a control unit for loading the program.
The present invention may also provide a device including a control unit incorporating a regression equation obtained by performing the method.
The present invention may provide a device including a control unit which loads a regression equation obtained by performing the method.
The present description includes the contents disclosed in Japanese Patent Application No. 2016-096681 from which this application claims priority.

Advantageous Effects of Invention

According to the present invention, the ratio of a measurement object substance to a comparison object substance can be more easily determined.

Brief Description of Drawings

Fig. 1A illustrates an example of the process from immunoreaction to absorbance measurement.
Fig. 1B schematically illustrates a technique for measuring the ratio of HbAlc to the total Hb according to a first embodiment of the present invention.
Fig. 1C is a functional block diagram of a configuration example of a measurement device for measuring the ratio of HbA1c to the total Hb.

Fig. 2 illustrates an example of the results of measuring, using a spectrophotometer, the absorption spectrum of a gold colloid agglutination due to an antigen-antibody reaction, where the horizontal axis shows wavelength and the vertical axis shows absorbance, the example showing an analysis result (measurement result) per amount of time elapsed.

Fig. 3 is a diagram obtained on the basis of the data of Fig. 2, illustrating the dependency of the ratio of absorbance at the wavelength of 525 nm and absorbance at the wavelength of 630 nm on the time elapsed from a reaction start time (time = 0).

Fig. 4 illustrates the relationship between a total Hb concentration y and an absorbance x.

Fig. 5 is a graph plotting, when a measurement was made using specimens in which the total Hb concentration was 0.5, 0.75, 1.0, 1.5, 2.0, and 2.5 mg/mL and the ratio of HbA1c to the total Hb was 3.7 to 15.3%, the ratio of HbA1c to the total Hb and the absorbance change (change amount $\Delta$) at the wavelength of 525 nm, five minutes after the start of a reaction.

Fig. 6 is a diagram in which the horizontal axis shows the total Hb concentration $\times$ the ratio of HbA1c to the total Hb = [$x_1$].

Fig. 7A is a diagram in which a concentration factor (Factor) is set such that the Factor becomes 1.00 when the total Hb concentration is 1.5 mg/mL, increases as the total Hb concentration decreases, and decreases as the total Hb concentration increases.

Fig. 7B is a diagram indicating the regression curve of Fig. 7A after the concentration factor (Factor) has been optimized for each total Hb concentration.

Fig. 8 shows the relationship, determined using the concentration factor shown in Fig. 7B, between [$x_2$] and absorbance change (change amount $\Delta$).

Fig. 9 shows a calibration curve by which the relationship between absorbance change and [$x_2$] is uniformly determined on the basis of Fig. 8 so as to be not dependent on the total Hb concentration.

Fig. 10 is a flowchart illustrating an example of processing according to the present embodiment.

Fig. 11A is a diagram continuing from Fig. 10.

Fig. 11B is a flowchart continuing from Fig. 11A and summarizing a measured value calculation procedure.

Fig. 12 is a flowchart illustrating an example of the processing in step S7 of Fig. 11A for determining the concentration factor (Factor).

Fig. 13 is a flowchart illustrating an example of the processing in step S7-4 of Fig. 12 for determining the concentration factor (Factor).

Fig. 14 is a graph plotting, when a measurement was made using specimens in which the total Hb concentration was 0.5, 0.75, 1.0, 1.5, 2.0, and 2.5 mg/mL, and the ratio of HbA1c to the total Hb was 3.7 to 15.3%, the ratio of HbA1c to the total Hb and the ratio of absorbance at the wavelength of 525 nm and absorbance at the wavelength of 630 nm, i.e., the absorbance ratio (Ratio), five minutes after the start of a reaction.

Fig. 15 is a diagram in which the horizontal axis shows the total Hb concentration $\times$ the ratio of HbA1c to the total Hb = [$x_1$].

Fig. 16A is a diagram in which the concentration factor (Factor) is set such that the Factor becomes 1.00 when the total Hb concentration is 1.5 mg/mL, increases as the total Hb concentration decreases, and decreases as the total Hb concentration increases.

Fig. 16B shows a regression curve of Fig. 16A after the concentration factor (Factor) has been optimized for each total Hb concentration.

Fig. 17 shows the relationship, determined using the concentration factor (Factor) shown in Fig. 16B, between [$x_2$] and the absorbance ratio (Ratio).

Fig. 18 shows a calibration curve obtained by exchanging the vertical axis and the horizontal axis of Fig. 17.

Fig. 19 is a graph according to a third embodiment of the present invention plotting, when a measurement was made using specimens in which the total Hb concentration was 1.0, 2.0, and 2.5 mg/mL and the ratio of HbA1c to the total Hb was 5.7 to 15.3%, the ratio of HbAlc to the total Hb and the absorbance change (change amount $\Delta$) at the wavelength of 525 nm, five minutes after the start of a reaction, where the substance for measurement having different concentrations had three types of dilution series.

Fig. 20 is a diagram in which the horizontal axis shows the total Hb concentration $\times$ the ratio of HbA1c to the total Hb = [$x_1$].

Fig. 21A is a diagram in which the concentration factor (Factor) is set such that the Factor becomes 1.00 when the Hb concentration is 2.0 mg/mL, increases as the total Hb concentration decreases, and decreases as the total Hb concentration increases.

Fig. 21B shows a regression curve of Fig. 21A after the concentration factor (Factor) has been optimized for each total Hb concentration.

Fig. 22 shows the relationship, determined using the concentration factor of Fig. 21B, between [$x_2$] and the absorb-

ance change (change amount Δ).

Fig. 23 shows a calibration curve by which the relationship between the absorbance change and [$x_2$] is uniformly determined on the basis of Fig. 22 so as to be not dependent on the total Hb concentration.

Description of Embodiments

The immunoassay refers to a method which utilizes an antigen-antibody reaction and of which the principle of detection may be an agglutination method or an agglutination inhibition method. The method may employ a carrier for carrying an antibody or an antigen. The antibody may be a monoclonal antibody or a polyclonal antibody. The carrier may be made of artificial carrier material or natural material that are generally used, including latex, gold colloid, and gelatin particles. In the case of the agglutination method, the absorbance change is such that an agglutination reaction occurs in the presence of a measurement object substance, resulting in an increase in absorbance. In the case of the agglutination inhibition method, an agglutination reaction is inhibited in the presence of a measurement object substance, whereby an increase in absorbance is suppressed. The ratio and degree of absorbance change may vary depending on the amount of the measurement object substance in the reaction system. Accordingly, in the present invention, an increase or decrease in absorbance due to a reaction utilizing the immunoassay can be utilized as the absorbance change amount.

The enzymatic method refers to the utilization of an enzyme and a color reagent to measure the measurement object, where a reaction is detected by measuring an absorbance specific to colored derivative which results from an enzyme reaction. The enzyme may include enzymes (such as proteinase, fructosyl peptide oxidase, and peroxidase) that are conventionally used during measurements for determining the ratio of HbA1c or glycoalbumin to the total hemoglobin or the total albumin. As a peroxidase substrate, any of generally used color reagents (such as 4-AA, DMA, OPD, ADOS, and TBHBA) may be used.

The absorbance change amount refers to an absorbance measured value at an absorption wavelength specific to a reaction caused in the case of the immunoassay or the enzymatic method utilized for measuring the measurement object. The absorbance change amount may be a change amount at a single wavelength (Δ value), or may be a difference between two wavelengths (main wavelength and sub-wavelength) or a ratio thereof (absorbance ratio of a main wavelength as the numerator and a sub-wavelength as the denominator; hereafter referred to as absorbance ratio or Ratio). An advantage of utilizing two wavelengths is that the influence of a drift phenomenon in the absorbance analysis device due to a voltage change on the absorbance measured value can be avoided.

The comparison object substance refers to a group of substances including the measurement object. For example, the comparison object substance refers to Hb with respect to HbA1c, albumin with respect to glycoalbumin, and, with respect to a specific type of isozyme, all isozymes for which the same catalyst reaction is performed.

The method for measuring the concentration of the comparison object substance may be a method which utilizes an absorbance change due to a reaction for measuring the comparison object substance. Examples include: in the case of Hb, a method which utilizes the measured value of absorbance unique to Hb (such as absorption wavelengths in the vicinity of 420 to 430 nm, 540 nm, and 570 nm); a method which utilizes the absorbance change amount due to a reaction by the immunoassay (such as agglutination method or agglutination inhibition method); and a method by which Hb is completely oxidized or reduced by means of processing using a surfactant, sodium azide, cyanide or the like, and then the measured value of unique absorbance in the oxidized or reduced state is utilized. In the case of albumin, as in the case of Hb, in addition to the method using the immunoassay, a classic method of utilizing the measured value of absorbance (colored derivative-specific absorption wavelength) using a die-binding method (which may be called a protein error method). The die-binding method is a method which utilizes a reaction in which, for example, bromcresol purple, bromcresol green, or a pH indicator develops color due to the presence of the protein irrespective of a pH change.

When there are two types of dilution series of the measurement object substance having different concentrations of the comparison object substance, the regression equation of the concentration factor can only become a straight line connecting two points, and the accuracy will become low. Conversely, if the number of types is increased, the accuracy of the regression equation of the concentration factor obtained by the present invention will increase. In this case, however, the labor required for the work to determine the absorbance change amount will increase and be impractical. Accordingly, in order to implement the present invention, at least three types, and more preferably at least five types, of dilution series of the measurement object substance having different concentrations of the comparison object substance may be prepared.

The concentration of the comparison object substance and the dilution series of the measurement object substance may be set for respective measurement ranges depending on the measurement object, or on the measurement system utilizing the immunoassay or the enzymatic method.

The inventors took intensive research and developed the present invention through the following procedure.

First, at different concentrations of the comparison object substance, a dilution series of the measurement object substance was prepared, and the absorbance change amount due to a reaction utilizing the immunoassay or enzymatic method was determined with respect to each dilution series.

Then, a calibration curve was created by plotting the dilution series of the measurement object substance obtained by measurement at each concentration of the comparison object substance on the x-axis, and the absorbance change amount on the y-axis. For example, in the case of the immunoagglutination method, the calibration curve becomes a sigmoid curve with an upper-right slope at each concentration of the comparison object substance. In this case, however, the calibration curve each independently exists merely as an individual calibration curve indicating the relationship between the dilution series of the measurement object substance and absorbance at each concentration of the comparison object substance, and do not enable the understanding of the relationship between the calibration curves.

Thus, a function was adopted in which the x-axis showed [x1] = the product of the concentration of the comparison object substance and the ratio of the measurement object substance to the comparison object substance, and the y-axis showed the absorbance change amount of the measurement object substance. In this way, it was learned that the relationship between the absolute amount of the measurement object substance and the absorbance change amount is clarified regardless of the concentration of the comparison object substance, and that a plurality of calibration curves, which in Fig. 5 each separately exist, exhibit the tendency to converge into a single calibration curve. That is, it was discovered that a first calibration curve having reduced dependency on the concentration of the comparison object substance can be obtained.

In other words, with respect to the calibration curve for each concentration of the comparison object substance, when the factor was set to an arbitrary integer, such as one, each calibration curve showed the tendency to converge into a single calibration curve.

This further led to the determination of a factor (hereafter referred to as a concentration factor (Factor) which was optimized with respect to the calibration curve for each concentration of the comparison object substance in such a way as to cause the calibration curves for each concentration of the comparison object substance to converge into a single calibration curve. Then, a function was adopted in which the x-axis was [x2] = the product of the concentration of the comparison object substance, the ratio of the measurement object substance to the comparison object substance, and the concentration factor determined for each concentration of the comparison object substance, and in which the y-axis showed the absorbance change amount. As a result, the plurality of calibration curves for each concentration of the comparison object substance converged into a single calibration curve. That is, it was discovered that it is possible to obtain a second calibration curve (multiplexed calibration curve) of which the dependency on the concentration of the comparison object substance was smaller than that of the first calibration curve.

Further, when the concentration factor for each concentration of the comparison object substance was plotted on the y-axis, and the concentration of the comparison object substance was plotted on the x-axis, it became possible to express the plots using a regression equation. By using the regression equation, it also became possible to determine a concentration factor (at total hemoglobin concentration) between plots having no actual measured values.

In addition, [x2] is the absorbance change amount [X]. The absorbance change amount [X] can be expressed by the relational expression [X] = (concentration of comparison object substance) × (ratio of measurement object substance to comparison object substance) × (concentration factor determined for each concentration of the comparison object substance). Accordingly, the relational expression can be further expressed by the following expression.

The ratio (%) of the measurement object substance to the comparison object substance = [X]/(concentration factor × concentration of comparison object substance) (1)

Thus, the ratio of the measurement object substance to the comparison object substance can be directly determined by obtaining the concentration of the comparison object substance, using the regression equation of the concentration factor determined in accordance with the present invention and expression (1), and measuring the absorbance change amount of the measurement object substance.

In the following, several embodiments of the present invention will be described with reference to the drawings. It should be noted, however, that the present invention is not limited to the specific examples described in the embodiments.

Further, the total Hb concentration, HbA1c, the absorbance change (change amount Δ), and the absorbance ratio (Ratio) are described as specific examples, and they respectively correspond to the broader concepts of concentration of comparison object substance, measurement object substance, and absorbance change amount. That is, the terms used in the following descriptions (including the drawings) are not intended to limit the present invention.

(First embodiment)

Fig. 1A illustrates an example of the process from immunoreaction to absorbance measurement. Referring to Fig. 1A(a), in a state in which an antibody is bound to a carrier, when an antigen as a sample is added, an antigen-antibody reaction is generated, as illustrated in Fig. 1A(b). As illustrated in Fig. 1A(c), in a state in which an immunoagglutination reaction is present, absorbance measurement is performed as illustrated in Fig. 1A(d). By utilizing such general antigen-antibody reaction and absorbance measurement, the HbA1c concentration can be similarly determined. While in the illustrated example, the carrier carries two types of antibody for reaction with one type of antigen, the antigen and the antibody may be reversed.

In the following, a method for determining the ratio of HbAlc to the total Hb will be described.

Fig. 1B schematically illustrates a technique for measuring the HbA1c concentration according to the first embodiment of the present invention. Fig. 1C is a functional block diagram of a configuration example of a device for measuring the HbA1c concentration.

In an exemplary implementation, as a reagent for determining the ratio of HbA1c to the total Hb, it is possible to use an HbA1c measurement reagent based on an agglutination reaction using gold colloids carrying an anti-HbA1c antibody and an anti-HbA0 antibody.

As illustrated in Fig. 1B, in the agglutination reaction using the gold colloids, a specimen 1 (blood) including HbA1c and an HbA1c measurement reagent 2 for generating a gold colloid agglutination reaction due to the anti-HbA1c antibody and the anti-HbA0 antibody are mixed to react with each other. The HbA1c measurement reagent 2 includes a gold colloid 3 carrying the anti-HbA1c antibody and a gold colloid 5 carrying the anti-HbA0 antibody. Using the specimen 1 and the HbA1c measurement reagent 2, generation of an aggregate 7 due to an antigen-antibody reaction with the HbA1c in the specimen 1 can be detected by an optical technique, for example.

It is known that gold colloids develop color by surface plasmon resonance of colloid particles, and that the color tone is changed by a change in particle size. This is utilized in the agglutination reaction using gold colloids to make it possible to observe and measure the generation of the aggregate 7 as a color tone change in a reaction solution (change from red to blue and finally to gold), i.e., as a temporal change in absorption spectrum.

As illustrated in Fig. 1C by way of example, an HbA1c concentration measurement device according to the present embodiment includes a light emitting unit 11, a reaction cell 15, a light reception unit 21, and a control unit 23 which controls the light emitting unit 11 and the light reception unit 21. A specimen 1 is irradiated with light emitted from the light emitting unit 11, and transmitted light is received by the light reception unit 21. On the basis of the relationship between light emission and light reception which are controlled by the control unit 23, the optical characteristics of the specimen 1 can be evaluated. In the present example, the light emitting unit 11 is provided with two LEDs 11a, 11b having different emission wavelengths. While spectrographic analysis is performed with reference to Fig. 2 and thereafter, it is preferable to use a simple device such as illustrated in Fig. 1C as an actual device.

The control unit 23 includes a first processing unit 23a (see Fig. 11B, for example) and a second processing unit 23b (see Fig. 12, for example). In a process for determining a concentration factor for each concentration of the comparison object substance in such a way that a plurality of calibration curves, which are obtained by determining the absorbance change amount of each dilution series of the measurement object substance due to the progress of a reaction between the dilution series of the measurement object substance, having different concentrations of the comparison object substance, and the measuring reagent, converge into a single calibration curve, the first processing unit 23a obtains

$$\text{the ratio (\%) of the measurement object substance to the comparison object substance} = [X]/(\text{concentration factor} \times \text{concentration of comparison object substance}) \qquad (1);$$

and

the second processing unit 23b determines a regression equation representing the relationship between the concentration factor for each concentration of the comparison object substance and the concentration of the comparison object substance.

The details of the processing by the first processing unit 23a and the second processing unit 23b will be described later.

The measurement device may be any device capable of performing absorbance measurement (spectrographic analysis device), and the light source is not limited to an LED or to any specific two wavelengths.

Fig. 2 illustrates an example of the results of measuring, using a spectrophotometer, the absorption spectrum of an antibody-carrying gold colloid agglutination reaction. The horizontal axis shows wavelength, and the vertical axis shows absorbance. Fig. 2 shows analysis results (measurement results) per amount of time elapsed.

As illustrated in Fig. 2, a decrease in absorbance was observed around the wavelength of 525 nm and an increase in absorbance was observed around 630 nm of the light with which a sample reacted using a specimen and a reagent was irradiated. The absorbance change is due to a gold colloid agglutination reaction, and the absorbance indicates the degree of agglutination.

The wavelength of around 525 nm is not a limitation and may include wavelengths in a wavelength range in which the absorbance decreases in the course of a temporal change in gold colloid agglutination reaction. The wavelength of around 630 nm is not a limitation and may include wavelengths in a wavelength range in which the absorbance increases in the course of the temporal change in gold colloid agglutination reaction.

Accordingly, on the basis of the absorbance change (at least one of decrease and increase), the amount of HbA1c can be determined. It should be noted, however, that when the change amount (absolute value) of the temporal change in absorbance is very small, a signal may be amplified using an amplifier and the like, so that the temporal change in absorbance can be accurately determined. Alternatively, a method which utilizes two wavelengths may be contemplated.

For example, Fig. 3 is a diagram obtained based on the data of Fig. 2. The diagram indicates the dependency, on the time elapsed from the reaction start time (time = 0), of the ratio of absorbance at the wavelength of 525 nm and absorbance at the wavelength of 630 nm, or absorbance525 nm/absorbance630 nm (hereafter referred to as "absorbance ratio (Ratio)"). As illustrated in Fig. 3, it is seen that the temporal decrease in absorbance (ratio of decrease in absorbance ratio (Ratio)) depends on the HbA1c concentration.

In this case, too, the wavelength of around 525 nm is not a limitation and may include wavelengths in a wavelength range in which the absorbance decreases in the course of the temporal change in gold colloid agglutination reaction. The wavelength of around 630 nm is not a limitation and may include wavelengths in a wavelength range in which the absorbance increases in the course of the temporal change in gold colloid agglutination reaction.

From Fig. 3, the temporal change in absorbance ratio (Ratio) can be noticeably observed. Accordingly, it can be understood that it is possible, using the absorbance ratio (Ratio), to easily measure the ratio of HbA1c, i.e., the measurement object, to the total Hb.

The ratio of HbA1c as the measurement object to the total Hb is defined by the ratio of a stable β-chain monoglycated Hb (Hb in which a single molecule of glucose is stably bound to a β-chain of Hb) to the total Hb included in red blood cells. Accordingly, in order to calculate the ratio of HbA1c to the total Hb, it is also necessary to calculate the total Hb concentration as the denominator.

Thus, a technique for measuring the ratio of HbA1c to the total Hb by taking the above into consideration will be described.

(Measurement of total Hb concentration after hemolytic dilution)

Fig. 10 is a flowchart illustrating an example of processing according to the present embodiment.

First, as specimen pretreatment, whole blood is lysed (process of causing red blood cells to collapse and to cause Hb to elute outside the blood cell) (step S1).

Then, a calibration curve for measuring the total Hb concentration is created as follows.

Calibrators that have been serially diluted using a pretreatment reagent used for hemolysis as a diluent, and that have known Hb concentrations (final concentration 0.5 to 2.5 mg/mL) are measured when absorbance at the wavelength of 405 nm is measured (step S2). The absorbance at (around) the absorption wavelength of 405 nm characteristic of Hb is on the horizontal axis, and the known total Hb concentrations are on the vertical axis.

Fig. 4 illustrates the relationship between the total Hb concentration (on the vertical axis (y)) and the absorbance (on the horizontal axis (x)). From this correlation diagram, it is possible to determine a calibration curve for measuring the total Hb concentration.

The calibration curve expressed by the following expression and illustrated in Fig. 4 is determined (step S3). In the present example, the calibration curve is a cubic regression line.

$$y = 201.67x^3 + 47.65x^2 + 15.059x - 0.3409,\ R^2 = 0.9973 \qquad (2)$$

With the calibration curve, it is possible to determine the total Hb concentration (step S4).

(Quantitative measurement of HbA1c)

The total Hb concentration can be measured through simple absorbance measurement. However, HbA1c quantitative measurement by an antigen-antibody reaction is performed in a quantitative balance between antigen and antibody. Further, as noted above, the concentration of HbA1c (i.e., the absolute amount of HbA1c in the measurement reaction system) also varies depending on the total Hb concentration, which is a variable value.
Thus, a measurement was made as follows, using specimens in which the total Hb concentration was 0.5, 0.75, 1.0, 1.5, 2.0, and 2.5 mg/mL, and in which the ratio of HbA1c to the total Hb was 3.7 to 15.3%.
Fig. 5 is a graph plotting the ratio of HbA1c to the total Hb and the absorbance change (change amount $\Delta$) at the wavelength of 525 nm, five minutes after the start of a reaction. As illustrated in Fig. 5, between the ratio of HbA1c to the total Hb and the absorbance change, dependency on the absolute amount of HbA1c in the measurement reaction system is recognized. It is seen that, as the absorbance change is dependent on the absolute amount of HbA1c in the measurement reaction system, different absorbance changes are exhibited depending on the total Hb concentration even at the same ratio of HbA1c to the total Hb.
In Fig. 5, the horizontal axis shows the ratio of HbA1c to the total Hb. As shown in Fig. 5, the absorbance change (change amount $\Delta$) with respect to the ratio of HbA1c to the total Hb is substantially proportional to the total Hb concentration in a predetermined HbA1c range (on the order of 6% to 12% in the figure).
From Fig. 5, the ratio of HbA1c to the total Hb is determined (Fig. 10, step S5).
As described above, in the present embodiment, it is possible, on the basis of the calibration curves shown in Fig. 5 which are dependent on the total Hb concentration, to determine the ratio of HbA1c depending on the absorbance change (change amount $\Delta$) to the total Hb.
As the calibration curves, it is also possible to use the calibration curves shown in Fig. 14, which will be described later.

(Second embodiment)

A second embodiment of the present invention will be described.
In the first embodiment, as illustrated in Fig. 5, it is necessary to set the calibration curves in an exhaustive manner for each total Hb concentration, and the processing for determining the HbA1c concentration becomes cumbersome.
In the following, a processing method that is performed in place of step S5 of the first embodiment in order to solve the above problem will be described.
In the present embodiment, a scheme based on an absorbance change (change amount $\Delta$), such as a temporal change in absorbance at the wavelength of 525 nm (see Fig. 2), will be described.
As described with reference to the first embodiment, Fig. 5 is a graph plotting the HbA1c concentration and the absorbance change (change amount $\Delta$) at the wavelength of 525 nm five minutes after the start of a reaction. As illustrated in Fig. 5, a relationship in which the absorbance change is dependent on the ratio of HbA1c to the total Hb is seen. It is seen that, as the absorbance change (change amount $\Delta$) is dependent on the HbA1c concentration (the absolute amount of HbA1c in the measurement reaction system), different absorbance changes are exhibited depending on the total Hb concentration even at the same ratio of HbA1c.
Fig. 11A is a diagram continuing from Fig. 10.
In Fig. 5, the horizontal axis shows the ratio of HbA1c to the total Hb. It is seen from Fig. 5 that the absorbance change (change amount $\Delta$) with respect to the ratio of HbA1c to the total Hb is substantially proportional to the total Hb concentration.
In Fig. 5, the relationship between the absorbance change (change amount $\Delta$) and the ratio of HbA1c is greatly dependent on the total Hb concentration. However, it has been learned that, as illustrated in Fig. 6, when the horizontal axis is the total Hb concentration $\times$ the ratio of HbAlc to the total Hb = $[x_1]$, the absorbance change (change amount $\Delta$) and $[x_1]$ exhibit the tendency to converge into a single relationship. The $[x_1]$ is the total Hb concentration $\times$ the ratio of HbA1c to the total Hb, and may be considered a first HbA1c concentration in which the total Hb concentration is taken into consideration.
It should be noted, however, that, as illustrated in Fig. 6, in a range of $[x_1]$ in which the absorbance change (change amount $\Delta$) is large, the influence of the total Hb concentration dependency is seen and the convergence is not very good. Thus, in order to improve the convergence of Fig. 6, i.e., in order that the relationship between the absorbance change (change amount $\Delta$) and $[x_1]$ has high correlation without depending on the total Hb concentration, a correction factor (concentration factor (Factor)) dependent on the total Hb concentration is employed.
Herein, as illustrated in Fig. 7A by way of example, the concentration factor (Factor) is set such that the Factor becomes 1.00 when the total Hb concentration is 1.50 mg/mL, increases as the total Hb concentration decreases, and decreases as the total Hb concentration increases (step S6).

With reference to Fig. 6, the horizontal axis is $[x_2]$ = the total Hb concentration × Factor × the ratio of HbA1c to the total Hb = concentration factor (Factor) × $[x_1]$. Then, the concentration factor (Factor) is determined for each total Hb concentration so that the relationships between the absorbance change (change amount Δ) and $[x_2]$ converge into a single relationship (step S7).

Then, as illustrated in Fig. 7B, it becomes possible to determine a concentration factor (Factor) that substantially monotonically decreases with respect to the Hb concentration.

Fig. 7B shows a regression curve of the total Hb concentration y and the concentration factor z. The regression curve can be expressed by the following regression equation. In this example, the regression equation is a cubic regression equation.

$$y = -0.1892z^3 + 1.014z^2 - 2.0729z + 2.492,\ R^2 = 0.9792 \qquad (3)$$

With this regression equation, it is possible to determine, from a total Hb concentration, the concentration factor at the total Hb concentration.

Fig. 8 shows the relationship, determined using the concentration factor (Factor) shown in Fig. 7B, between $[x_2]$ and the absorbance change (change amount Δ). As illustrated in Fig. 8, it is possible to determine, in the entire range of $[x_2]$, the relationship between $[x_2]$ and the absorbance change (change amount Δ) that is not dependent on the total Hb concentration.

The $[x_2]$ is the total Hb concentration × the ratio of HbA1c to the total Hb × the concentration factor (Factor), and may be considered the ratio of a second HbAlc to the total Hb in which the total Hb concentration and the concentration factor therefor are taken into consideration.

When the x-axis and the y-axis of Fig. 8 are exchanged, it becomes possible to determine, as illustrated in Fig. 9, the relationship between the absorbance change (change amount Δ) and $[x_2]$ uniformly without being dependent on the total Hb concentration (step S8).

That is, using the calibration curve shown in Fig. 9, it becomes possible to determine the ratio of HbA1c to the total Hb according to the expression (4) below, through simpler than conventional processing. Thus, $[x_2]$ is the absorbance change amount [X].

[Expression 1]

$$\mathrm{HbA1c}(\%) = \frac{[X]}{\mathrm{Factor} \times \mathrm{Total\ Hb\ Concentration}} \qquad (4)$$

As described above, according to the present embodiment, it is possible to easily determine the HbA1c concentration using a single calibration curve.

The concentration factor (Factor) is dependent on the reagent. Accordingly, when a reagent from a different lot is used, it is preferable to newly determine the relationship of Fig. 7B.

Fig. 12 and Fig. 13 are flowcharts illustrating an example of the processing in S7 of Fig. 11A for determining the concentration factor (Factor).

First, in step S7-1, absorbance is measured with respect to a dilution series (diluted samples) of HbA1c for each of different total Hb concentrations.

In step S7-2, with respect to all dilution series, an absorbance change (change amount Δ) or an absorbance ratio (Ratio), which will be described later with reference to the third embodiment, is determined from the measured absorbance as the absorbance change.

In step S7-3, all of the concentration factors (Factor) for the different total Hb concentrations are fixed at arbitrary integers, and $R^2$ of $[x_2]$ and the absorbance change amount, or the absorbance ratio (Ratio), is determined.

In step S7-4, the concentration factor (Factor) is optimized for each of different total Hb concentrations.

The procedure for optimization will be described with reference to Fig. 13 by way of example.

In step S7-5, an n-order regression equation for the total Hb concentration and the concentration factor (Factor) is created. The corresponding figure is Fig. 7B, for example.

Referring to Fig. 13, the processing in step S7-4 for optimizing the concentration factor (Factor) for each of different total Hb concentrations will be described.

In Fig. 13, the first round where n = 1 is as follows.

Step S21-1: The concentration factors (Factor) for the respective concentrations other than the total Hb concentration of the optimization object are fixed to arbitrary integers, and a concentration factor (Factor) that maximizes $R^2$ of [x] and the absorbance change (change amount $\Delta$) or the absorbance ratio (Ratio) is determined at the total Hb concentration of the optimization object.

Step S21-2: Using the concentration factor (Factor) determined in step S21-1 at the concentration considered the optimization object, and with the concentration factors (Factor) at the respective remaining concentrations, except for a concentration newly considered an optimization object, fixed to the arbitrary integers used in step S21-1, a concentration factor (Factor) that maximizes $R^2$ of [x] and the absorbance change (change amount $\Delta$) or the absorbance ratio (Ratio) is determined at the total Hb concentration of the optimization object.

Step S21-3: Using the Factors determined in steps S21-1, 21-2 at the respective concentrations that were considered the optimization objects, and with the Factors at the respective remaining concentrations, except for a concentration that is newly considered an optimization object, fixed to the arbitrary integers used in step S21-1, a concentration factor (Factor) that maximizes $R^2$ of [x] and the absorbance change (change amount $\Delta$) or the absorbance ratio (Ratio) is determined at the total Hb concentration of the optimization object.

Step S21-4 and thereafter: With respect to the remaining concentrations, using the concentration factor (Factor) obtained in the previous step, a concentration factor (Factor) that maximizes $R^2$ of [x] and the absorbance change (change amount $\Delta$) or the absorbance ratio (Ratio) is determined successively and similarly at each concentration.

In the second round and thereafter, i.e., when $n \geq 2$, the following processing is performed.

Step S22-1: Using, as the concentration factor (Factor) at the respective concentrations other than the total Hb concentration of the optimization object, the respective concentration factors (Factor) determined in the previous round, a concentration factor (Factor) that maximizes $R^2$ of [x] and the absorbance change (change amount $\Delta$) or the absorbance ratio (Ratio) is determined at the total Hb concentration of the optimization object.

Step S22-2: Using the concentration factor (Factor) determined in step S22-1 at the concentration considered the optimization object, and using, as the concentration factors (Factor) at the respective remaining concentrations except for a concentration that is newly considered an optimization object, the respective concentration factors (Factor) determined in the previous round, a concentration factor (Factor) that maximizes $R^2$ of [x] and the absorbance change (change amount $\Delta$) or the absorbance ratio (Ratio) is determined at the total Hb concentration of the optimization object.

Step S22-3: Using the concentration factor (Factor) determined at the respective concentrations that were considered the object in step S22-1 and step S22-2, and using, as the respective concentration factors (Factor) at the respective concentrations except for a concentration that is newly considered an optimization object, the concentration factors (Factor) at the respective concentrations determined in the previous round, a concentration factor (Factor) that maximizes $R^2$ of [x] and the absorbance change (change amount $\Delta$) or the absorbance ratio (Ratio) is determined at the total Hb concentration of the optimization object.

Step S22-4 and thereafter: With respect to the remaining concentrations, using the concentration factor (Factor) obtained in the previous step, a concentration factor (Factor) that maximizes $R^2$ of [x] and the absorbance change (change amount $\Delta$) or the absorbance ratio (Ratio) is determined successively and similarly at each concentration.

The processing from step S22-1 to step S22-4 is repeated.

In step S23, as the final step, if the concentration factor (Factor) at each total Hb concentration becomes the same value as that of the previous round, the Factor optimization processing ends.

As described above, the optimization processing for the concentration factor (Factor) is performed for each of different total Hb concentrations, whereby it becomes possible to determine the relationship in which the concentration factor (Factor) is dependent on the Hb concentration, as illustrated in Fig. 7B. A scheme other than the optimization schemes illustrated in Figs. 12 and 13 may be utilized provided that the scheme involves processing by which the concentration factor (Factor) can be optimized for each of different total Hb concentrations.

In the following, the measured value calculation procedure is summarized.

Fig. 11B continues from Fig. 11A and is a flowchart summarizing the measured value calculation procedure.

1) The total Hb concentration is measured (calibration curve: absorbance 405 nm vs total Hb concentration) (step S11).

2) The absorbance change amount (in the present embodiment, an absorbance change (change amount $\Delta$)) due to a gold colloid agglutination reaction in the sample including HbA1c is measured (step S12).

3) The Factor is calculated from the total Hb concentration obtained in 1) (step S11) and the regression equation (3) expressing the relationship between the total Hb concentration and the concentration factor (calibration curve: total Hb concentration vs Factor) (step S13).

4) The relevant values are substituted into the expression (4), and the ratio of HbA1c to the total Hb is calculated (step S14).

In this way, according to the present embodiment, it is possible to easily determine, using a single calibration curve (multiplexed calibration curve) that is not dependent on the total Hb concentration, the ratio of HbA1c to the total Hb from the absorbance measured value.

As described above, the ratio of HbA1c to the total Hb vs the absorbance change amount exhibits an independent curve for each total Hb concentration. However, according to the present embodiment, when the total Hb concentration-dependent factor (Factor) is set, the absorbance change amount vs [X] converges into a single curve, irrespective of the total Hb concentration. Accordingly, it becomes possible to measure the ratio of HbA1c to the total Hb without creating a number of calibration curves for each total Hb concentration in an exhaustive manner.

(Third embodiment)

A third embodiment of the present invention will be described.

In the present embodiment, the calibration curve for determining the HbA1c concentration is determined using, as an indicator of absorbance change, an absorbance ratio (absorbance 525 nm/absorbance 630 nm) that is a variable.

Fig. 14 is a graph plotting, when a measurement was made using specimens in which the total Hb concentration was 0.5, 0.75, 1.0, 1.5, 2.0, and 2.5 mg/mL and the ratio of HbA1c to the total Hb was 3.7 to 15.3%, the ratio of HbA1c to the total Hb, and the ratio of absorbance at the wavelength of 525 nm and absorbance at the wavelength of 630 nm, i.e., the absorbance ratio (Ratio), five minutes after the start of a reaction.

In Fig. 14, the horizontal axis shows the ratio of HbA1c to the total Hb. As shown in Fig. 14, the absorbance ratio (Ratio) with respect to the ratio of HbA1c to the total Hb is substantially inversely proportional to the total Hb concentration.

Thus, as shown in Fig. 15, the horizontal axis is made the total Hb concentration × the ratio of HbA1c to the total Hb = [$x_1$]. In this way, it has been learned that, while in Fig. 5 the relationship between the absorbance ratio (Ratio) and the ratio of HbA1c to the total Hb is greatly dependent on the total Hb concentration, the absorbance ratio (Ratio) and [$x_1$] exhibit the tendency to converge into a single relationship. The [$x_1$] is the total Hb concentration × the ratio of HbA1c to the total Hb, and may be considered the first ratio of a HbA1c to the total Hb in which the total Hb concentration is taken into consideration.

It should be noted, however, that, as illustrated in Fig. 15, in a [$x_1$] range in which the change in the absorbance ratio (Ratio) is large, the influence of the total Hb concentration dependency is seen and the convergence is not very good. Thus, in order to improve the convergence of Fig. 15, i.e., in order that the relationship between the absorbance ratio (Ratio) and [$x_1$] has high correlation without depending on the total Hb concentration, a correction factor (concentration factor (Factor)) dependent on the total Hb concentration is employed, similarly to the second embodiment.

Herein, as illustrated in Fig. 16A by way of example, the concentration factor (Factor) is set such that the Factor becomes 1.00 when the total Hb concentration is 1.50 mg/mL, increases as the total Hb concentration decreases, and decreases as the total Hb concentration increases (Fig. 11A, step S6).

Then, with reference to Fig. 15, the horizontal axis is made [$x_2$] = total Hb concentration × Factor × the ratio of HbA1c to the total Hb = concentration factor (Factor) × [$x_1$]. Then, the concentration factor (Factor) is determined such that the relationship between the absorbance ratio (Ratio) and [$x_2$] converges into a single relationship (Fig. 11A, step S7).

Accordingly, as illustrated in Fig. 16B, the concentration factor (Factor) that monotonically decreases with respect to the Hb concentration can be determined.

Fig. 16B shows a regression curve of the total Hb concentration y and the concentration factor z. The regression curve can be expressed by the following regression equation, which in this example is a cubic regression equation.

$$y = -0.0871z^3 + 0.5819z^2 - 1.508z + 2.2185,\ R^2 = 0.9984 \qquad (5)$$

With this regression equation, it is possible to determine, from a total Hb concentration, the concentration factor at the total Hb concentration.

Fig. 17 shows the relationship, determined using the concentration factor (Factor) shown in Fig. 16B, between [$x_2$] and the absorbance ratio Ratio. As illustrated in Fig. 17, it is possible to determine, in the entire range of [$x_2$], the relationship between [$x_2$] and the absorbance ratio (Ratio) that is not dependent on the total Hb concentration.

The [$x_2$] is the total Hb concentration × the ratio of HbA1c to the total Hb × concentration factor (Factor), and may be considered the second ratio of a HbA1c to the total Hb in which the total Hb concentration and the concentration factor therefor are taken into consideration.

When the x-axis and the y-axis of Fig. 17 are exchanged, it becomes possible to determine, as illustrated in Fig. 18, the relationship between the absorbance ratio (Ratio) and [$x_2$] uniformly without being dependent on the total Hb concentration (Fig. 11A, step S8).

That is, using the calibration curve shown in Fig. 18, it is possible to determine the HbA1c concentration according to an expression (6) below through simpler than conventional processing. The [$x_2$] is namely the absorbance change amount [X].

[Expression 2]

$$\mathrm{HbA1c}(\%) = \frac{[X]}{\mathrm{Factor} \times \mathrm{Total\ Hb\ Concentration}} \qquad (6)$$

In the present embodiment, the absorbance ratio (Ratio) that is an absorbance change amount is used as a parameter. Accordingly, even when the absorbance change amount is small, it is possible to create the calibration curve by increasing the change amount. It is also possible to accurately measure the ratio of HbA1c to the total Hb.

1) The total Hb concentration is measured (calibration curve: absorbance 405 nm vs total Hb concentration) (Fig. 11B, step S11).
2) The absorbance ratio (Ratio) is measured as the absorbance change amount due to a gold colloid agglutination reaction in a sample including HbA1c (Fig. 11B, step S12).
3) The Factor is calculated from the total Hb concentration obtained in 1) (Fig. 11B, step S11) and the regression equation (5) expressing the relationship between the total Hb concentration and the concentration factor (calibration curve: total Hb concentration vs Factor) (Fig. 11B, step S13).
4) The relevant values are substituted into the expression (6), and the ratio of HbA1c to the total Hb is calculated (Fig. 11B, step S14).

In this way, according to the present embodiment, using a single calibration curve (multiplexed calibration curve) that is not dependent on the total Hb concentration, it becomes possible to easily determine the ratio of HbA1c to the total Hb from the absorbance measured value.
As described above, the ratio of HbA1c to the total Hb vs the absorbance change amount exhibits an independent curve for each total Hb concentration. However, according to the present embodiment, when the total Hb concentration-dependent factor (Factor) is set, the absorbance change amount vs [X] converges into a single curve irrespective of the total Hb concentration. Accordingly, it becomes possible to measure the ratio of HbA1c to the total Hb without creating a number of calibration curves for each total Hb concentration in an exhaustive manner.

(Fourth embodiment)

Similarly to the second embodiment, in the fourth embodiment of the present invention, a scheme will be described which is based on the absorbance change (change amount Δ), such as a temporal change in absorbance at the wavelength of 525 nm (see Fig. 2). The example includes three types of dilution series of the substances for measurement having different concentrations.
Fig. 19 is a graph plotting, when a measurement was made using specimens in which the total Hb concentration was 1.0, 2.0, and 2.5 mg/mL and the ratio of HbA1c to the total Hb was 5.7 to 15.3%, the ratio of HbA1c to the total Hb and the absorbance change (change amount Δ) at the wavelength of 525 nm, five minutes after the start of a reaction.
In Fig. 19, the horizontal axis shows the ratio of HbA1c to the total Hb. It is seen from Fig. 19 that the absorbance change (change amount Δ) with respect to the ratio of HbA1c to the total Hb is substantially proportional to the total Hb concentration.
Thus, the horizontal axis is made the total Hb concentration × the ratio of HbA1c to the total Hb = [$x_1$], as shown in Fig. 20. In this way, it has been learned that, while the relationship between the absorbance change (change amount Δ) and the ratio of HbA1c to the total Hb is greatly dependent on the total Hb concentration in Fig. 19, the absorbance change (change amount Δ) and [$x_1$] exhibit the tendency to converge into a single relationship. The [$x_1$] is the total Hb concentration × the ratio of HbA1c to the total Hb, and may be considered the first ratio of a HbA1c to the total Hb in which the total Hb concentration is taken into consideration.
It should be noted, however, that, as illustrated in Fig. 20, in a [$x_1$] range in which the absorbance change (change amount Δ) is large, the influence of the total Hb concentration dependency is seen and the convergence is not very good. Thus, in order to improve the convergence of Fig. 20, i.e., in order that the relationship between the absorbance change (change amount Δ) and [x1] has high correlation without depending on the total Hb concentration, a correction factor (concentration factor (Factor)) dependent on the total Hb concentration is employed, similarly to the second and third embodiments.
Herein, as illustrated in Fig. 21A by way of example, the concentration factor (Factor) is set such that the Factor becomes 1.00 when the total Hb concentration is 2.00 mg/mL, increases as the total Hb concentration decreases, and decreases as the total Hb concentration increases (Fig. 11A, step S6).

With reference to Fig. 20, the horizontal axis is made $[x_2]$ = the total Hb concentration × Factor × the ratio of HbA1c to the total Hb = the concentration factor (Factor) × $[x_1]$. Then, the concentration factor (Factor) is determined such that the relationship between the absorbance change (change amount Δ) and $[x_2]$ converges into a single relationship (Fig. 11A, step S7).

In this way, as illustrated in Fig. 21B, a concentration factor (Factor) which monotonically decreases with respect to the Hb concentration can be determined.

Fig. 21B shows a regression curve of the total Hb concentration y and concentration factor z. The regression curve can be expressed by the following regression equation. In this example, the regression equation is a second-order regression equation.

$$y = 0.18z^2 - 0.93z + 2.18,\ R^2 = 1 \qquad (7)$$

With this regression equation, it is possible to determine, from a total Hb concentration, the concentration factor at the total Hb concentration.

Fig. 22 shows the relationship, determined using the concentration factor (Factor) of Fig. 21B, between $[x_2]$ and the absorbance change (change amount Δ). As illustrated in Fig. 22, it is possible to determine, in the entire range of $[x_2]$, the relationship between $[x_2]$ and the absorbance change (change amount Δ) that is not dependent on the total Hb concentration.

The $[x_2]$ is the total Hb concentration × the ratio of HbA1c to the total Hb × the concentration factor (Factor), and may be considered the second ratio of a HbAlc to the total Hb in which the total Hb concentration and the concentration factor therefor are taken into consideration.

When the x-axis and the y-axis in Fig. 22 are exchanged, as illustrated in Fig. 23, it becomes possible to determine the relationship between the absorbance change (change amount Δ) and $[x_2]$ uniformly without being dependent on the total Hb concentration (Fig. 11A, step S8).

That is, using the calibration curve shown in Fig. 22, it is possible to determine the HbA1c concentration according to an expression (8) below through simpler than conventional processing. The $[x_2]$ is namely the absorbance change (change amount Δ) [X].

[Expression 3]

$$\mathrm{HbA1c}(\%) = \frac{[X]}{\mathrm{Factor} \times \mathrm{Total\ Hb\ Concentration}} \qquad (8)$$

In this way, according to the present embodiment, using a single calibration curve (multiplexed calibration curve) that is determined using three types of dilution series of the substances for measurement having different concentrations and that is not dependent on the total Hb concentration, it is possible to easily determine the ratio of HbA1c to the total Hb from the absorbance measured value.

1) The total Hb concentration is measured (calibration curve: absorbance 405 nm vs total Hb concentration) (Fig. 11B, step S11).
2) The absorbance change amount (in the present embodiment, the absorbance change (change amount Δ)) due to a gold colloid agglutination reaction in a sample including HbA1c is measured (Fig. 11B, step S12).
3) The Factor is calculated from the total Hb concentration obtained in 1) (Fig. 11B, step S11), and the regression equation (7) expressing the relationship between the total Hb concentration and the concentration factor (calibration curve: total Hb concentration vs Factor) (Fig. 11B, step S13).
4) The relevant values are substituted into the expression (8), and the ratio of HbAlc to the total Hb is calculated (Fig. 11B, step S14).

As described above, the ratio of HbA1c to the total Hb vs the absorbance change amount exhibits an independent curve for each total Hb concentration. However, according to the present embodiment, when the total Hb concentration-dependent factor (Factor) is set, the absorbance change amount vs [X] converges into a single curve irrespective of the total Hb concentration. Accordingly, it becomes possible to measure the ratio of HbA1c to the total Hb without creating a number of calibration curves for each total Hb concentration in an exhaustive manner.

Processing and controls may be implemented by means of software processing using a central processing unit (CPU) or a graphics processing unit (GPU), or by means of hardware processing using an application specific integrated circuit (ASIC) or a field programmable gate array (FPGA).

In the foregoing embodiments, the configurations and the like illustrated in the attached drawings are not limitations and may be modified, as appropriate, within the range in which the effects of the present invention can be exerted. The embodiments may be modified, as appropriate, and implemented without departing from the scope of the objective of the present invention.

The constituent elements of the present invention may be optionally selected, and an invention provided with selected configurations is also included in the present invention.

For example, an absorbance measurement unit and a control unit may comprise an integrated device or may comprise separate (independent) devices.

The control unit may incorporate a program for implementing a method for determining the ratio of a measurement object substance to a comparison object substance, or a regression equation obtained using the method for determining the ratio of a measurement object substance to a comparison object substance. The control unit may execute the program or the regression equation loaded from outside of a device.

For example, a portable examination terminal (examination device) including the light emitting unit 11, the reaction cell 15, and the light reception unit 21 shown in Fig. 1C may be formed separately from the control unit. A measured value obtained by means of the examination terminal may be sent, via a wired or wireless communication unit, not shown, to the control unit 23 provided at a management center and the like, and an examination result may be obtained on the management center side. Alternatively, the examination result may be returned to the examination terminal.

In this way, it becomes possible to perform examination of a specimen only using the examination terminal (examination device).

Information of a regression equation may vary from one production lot to another of a reagent contained in the measurement (assay) chip. Accordingly, the information of the regression equation obtained in advance using the method of the present invention may be stored in the measurement (assay) chip.

A program for achieving the functions described in the foregoing embodiments may be recorded in a computer-readable recording medium, and the program recorded in the recording medium may be loaded into a computer system and executed to perform the processing in various units. The "computer system" herein includes hardware, such as an OS and a peripheral device.

Industrial Applicability

The present invention may be utilized as a method for determining the ratio of HbAlc, glycoalbumin, isozyme or the like to a comparison object substance.

Reference Signs List

1 Specimen
2 HbAlc measurement reagent
3 Anti-HbAlc antibody-sensitized gold colloid
5 Anti-HbAO antibody-sensitized gold colloid
7 Aggregate
11 Light emitting unit
15 Reaction cell
21 Light reception unit
23 Control unit

All of the publications, patents, and patent applications cited in the present description are incorporated herein by reference.

## Claims

**1.** A method for determining a ratio of a measurement object substance to a comparison object substance using a measuring reagent for the measurement object substance,
the method comprising, in a process for determining, at each concentration of the comparison object substance, a concentration factor such that a plurality of calibration curves, which are obtained by determining an absorbance change amount of each dilution series of the measurement object substance due to the progress of a reaction between the dilution series of the measurement object substance having different concentrations of the comparison object substance and the measuring reagent, converge into a single calibration curve:

a step of obtaining a ratio (%) of the measurement object substance to the comparison object substance = [X]/(concentration factor $\times$ concentration of comparison object substance) (1); and
a step of determining a regression equation expressing a relationship between the concentration factor for each concentration of the comparison object substance and the concentration of the comparison object substance.

**2.** The method according to claim 1, wherein the step of determining the absorbance change amount comprises:

a step of determining a change amount of an increase or decrease in absorbance at a characteristic single wavelength due to the progress of a reaction caused by a specimen including the measurement object substance and the measuring reagent; or
a step of determining a change amount in an increase or decrease in absorbance at characteristic two wavelengths due to the progress of a reaction caused by the specimen including the measurement object substance and the measuring reagent, the change amount being a difference or ratio between a first absorbance at a first wavelength at which the absorbance increases or decreases and a second absorbance at a second wavelength at which the absorbance increases or decreases.

**3.** The method according to claim 1 or 2, wherein:

the measuring reagent is a measuring reagent utilizing any of an immunoassay including an agglutination method and an agglutination inhibition method, or an enzymatic method,
the method for determining the ratio comprising a step of determining the ratio of the measurement object substance to the comparison object substance from: the expression (1); a regression equation of the concentration factor optimized for each concentration of the comparison object substance and the concentration of the comparison object substance; and an absorbance change amount obtained by measuring a specimen including the measurement object substance using the measuring reagent.

**4.** A program for causing a computer to perform the method according to any one of claims 1 to 3.

**5.** A computer-readable recording medium having the program of claim 4 recorded thereon.

**6.** A device for determining a ratio of a measurement object substance to a comparison object substance using a measuring reagent for the measurement object substance,
the device comprising:

a control unit for performing the method according to any one of claims 1 to 3; and
an absorbance measurement unit,
wherein the control unit and the absorbance measurement unit are integrally or separately provided.

**7.** A device comprising a control unit incorporating the program according to claim 4.

**8.** A device comprising a control unit for loading the program according to claim 4.

**9.** A device comprising a control unit incorporating a regression equation obtained by performing the method according to any one of claims 1 to 3.

**10.** A device comprising a control unit for loading a regression equation obtained by performing the method according to any one of claims 1 to 3.

Fig. 1A

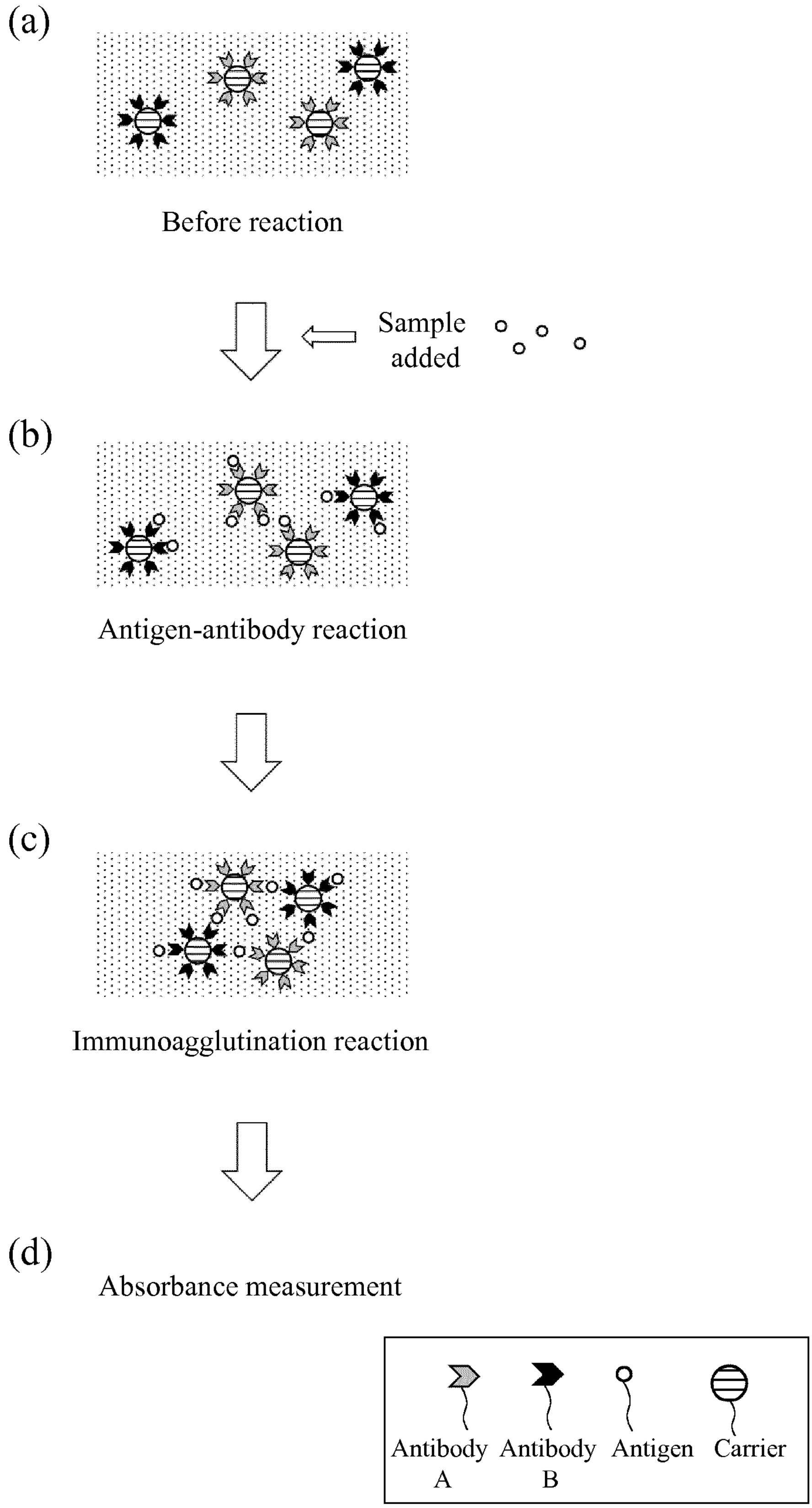
(a)
Before reaction
Sample added
(b)
Antigen-antibody reaction
(c)
Immunoagglutination reaction
(d)
Absorbance measurement
Antibody A
Antibody B
Antigen
Carrier

Fig. 1B

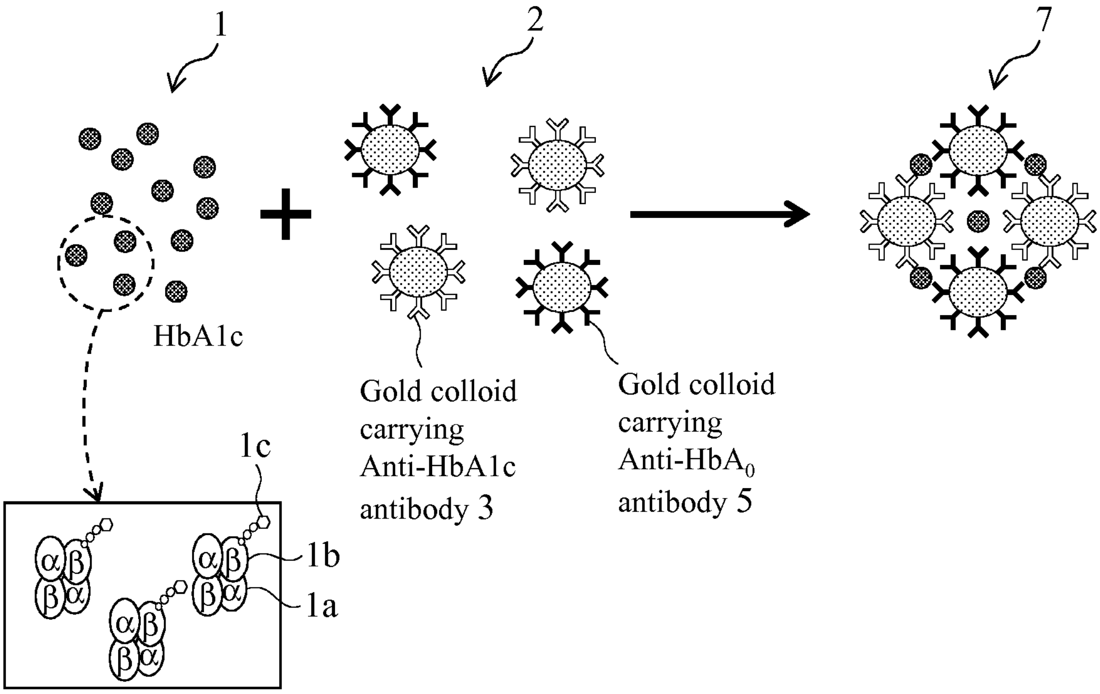
1
2
7
HbA1c
1c
1b
1a
Gold colloid carrying Anti-HbA1c antibody 3
Gold colloid carrying Anti-HbA0 antibody 5

Fig. 1C

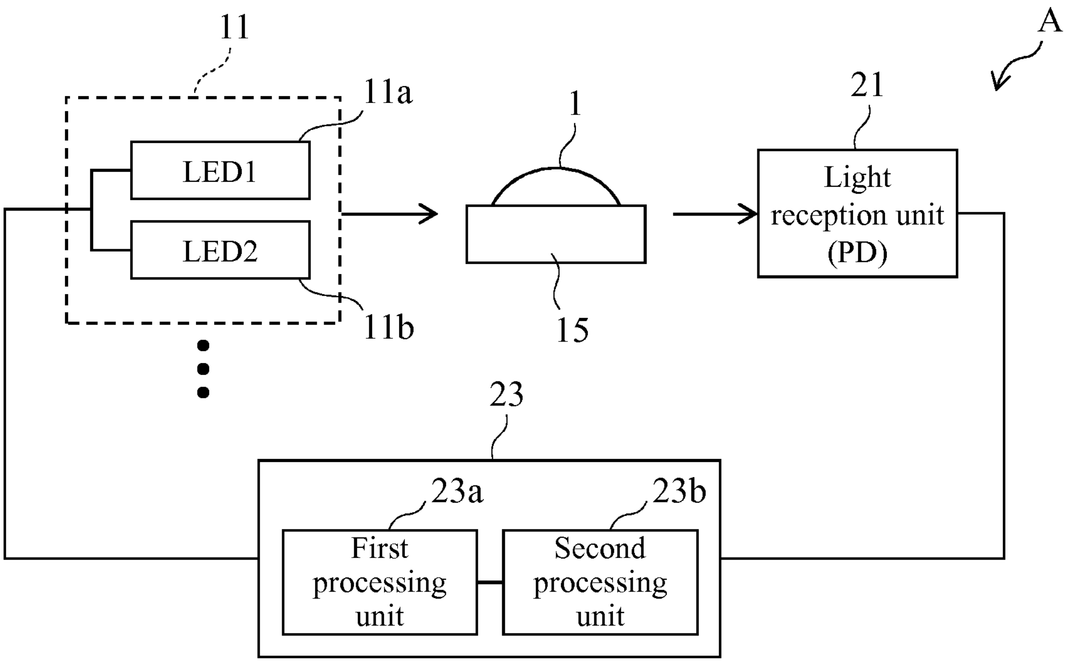
11
11a
LED1
LED2
11b
1
15
21
Light reception unit (PD)
A
23
23a
23b
First processing unit
Second processing unit

Fig. 2

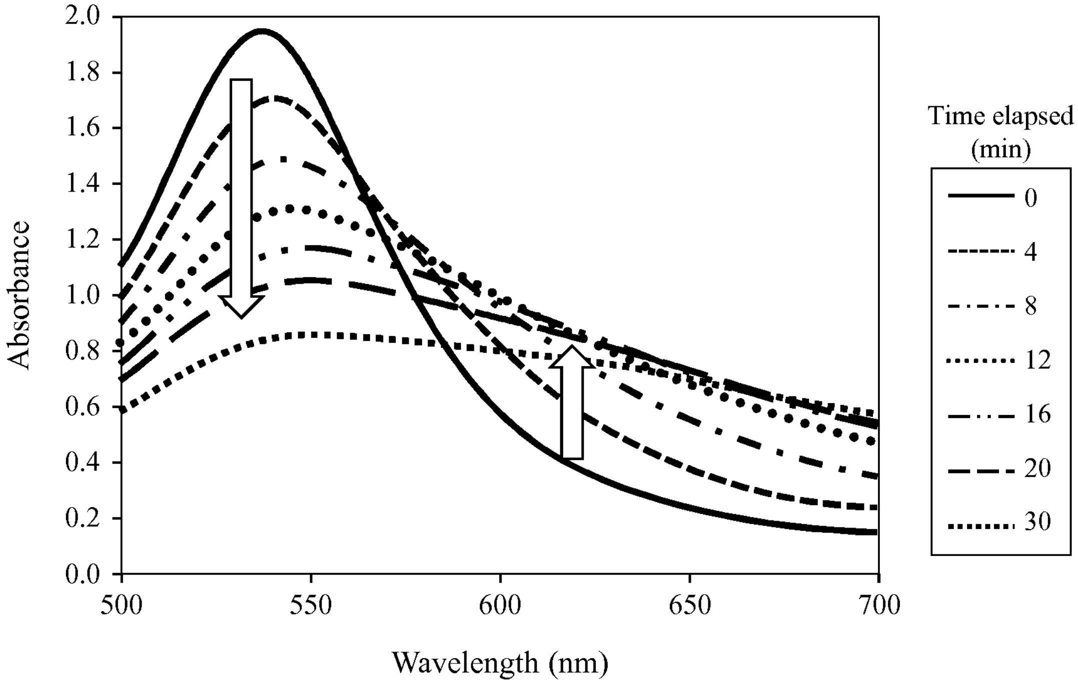
2.0
1.8
1.6
1.4
1.2
1.0
0.8
0.6
0.4
0.2
0.0
Absorbance
500
550
600
650
700
Wavelength (nm)
Time elapsed (min)
0
4
8
12
16
20
30

Fig. 3

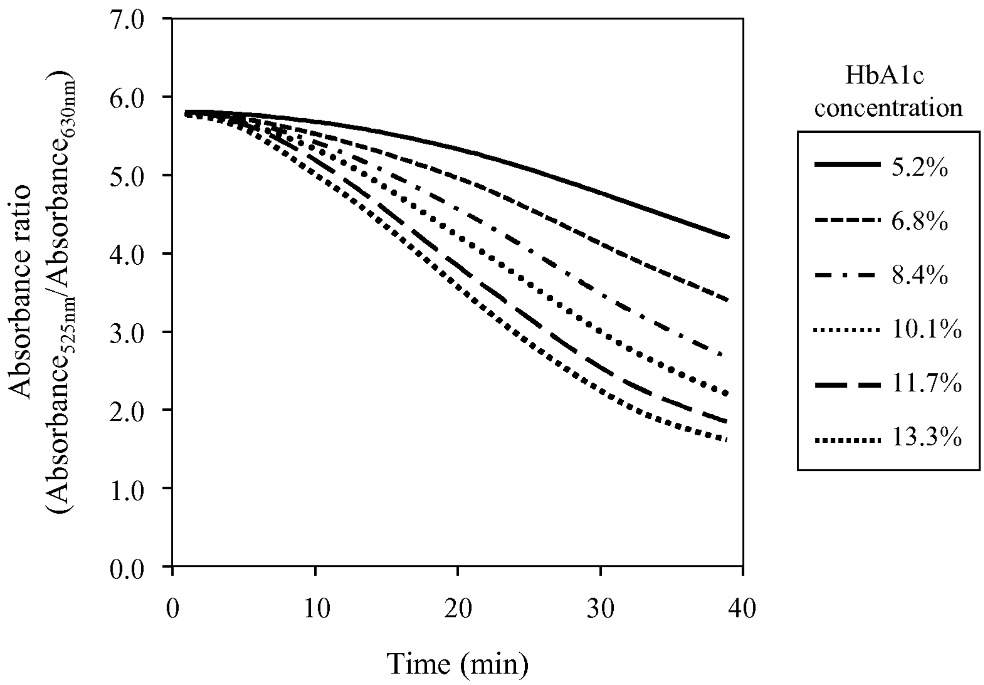
7.0
6.0
5.0
4.0
3.0
2.0
1.0
0.0
Absorbance ratio
(Absorbance525nm/Absorbance630nm)
0
10
20
30
40
Time (min)
HbA1c concentration
5.2%
6.8%
8.4%
10.1%
11.7%
13.3%

Fig. 4

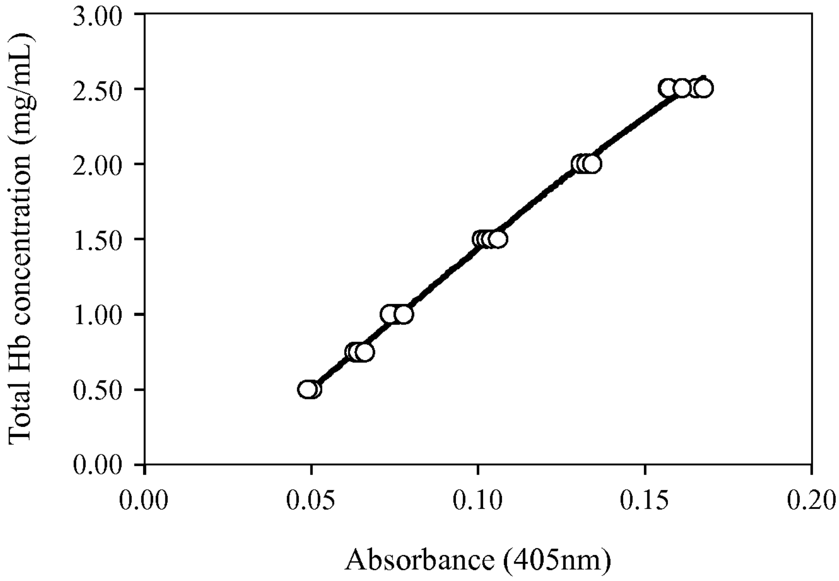
3.00
2.50
2.00
1.50
1.00
0.50
0.00
Total Hb concentration (mg/mL)
0.00
0.05
0.10
0.15
0.20
Absorbance (405nm)

Fig. 5

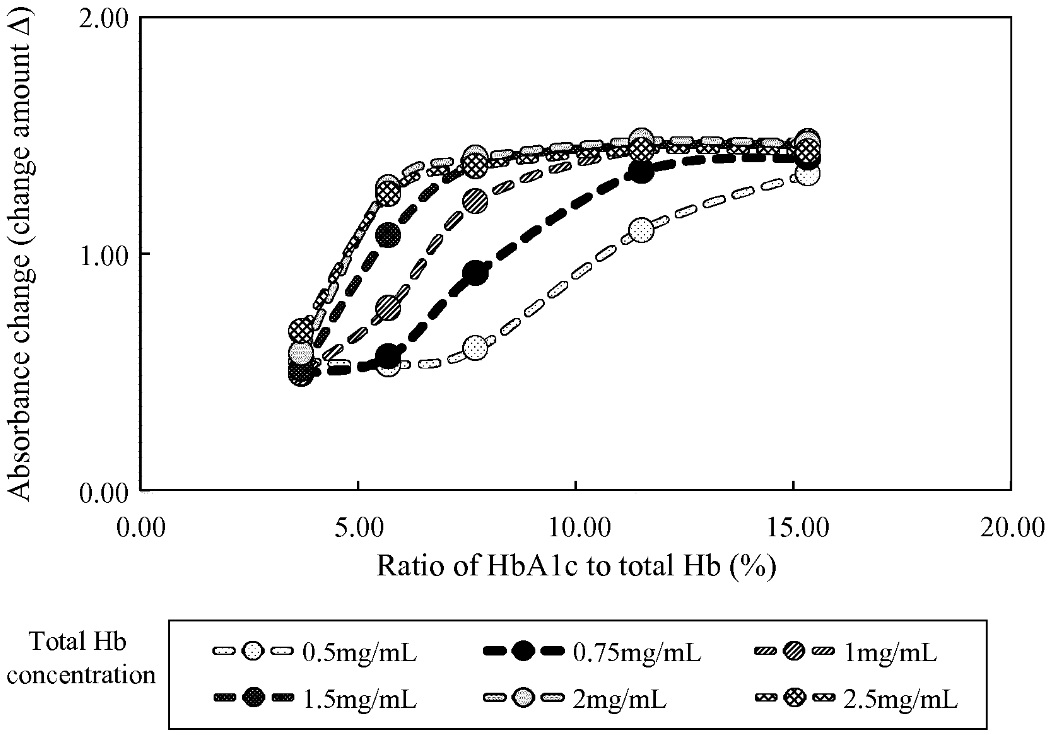
2.00
1.00
0.00
Absorbance change (change amount Δ)
0.00
5.00
10.00
15.00
20.00
Ratio of HbA1c to total Hb (%)
Total Hb concentration
0.5mg/mL
0.75mg/mL
1mg/mL
1.5mg/mL
2mg/mL
2.5mg/mL

Fig. 6

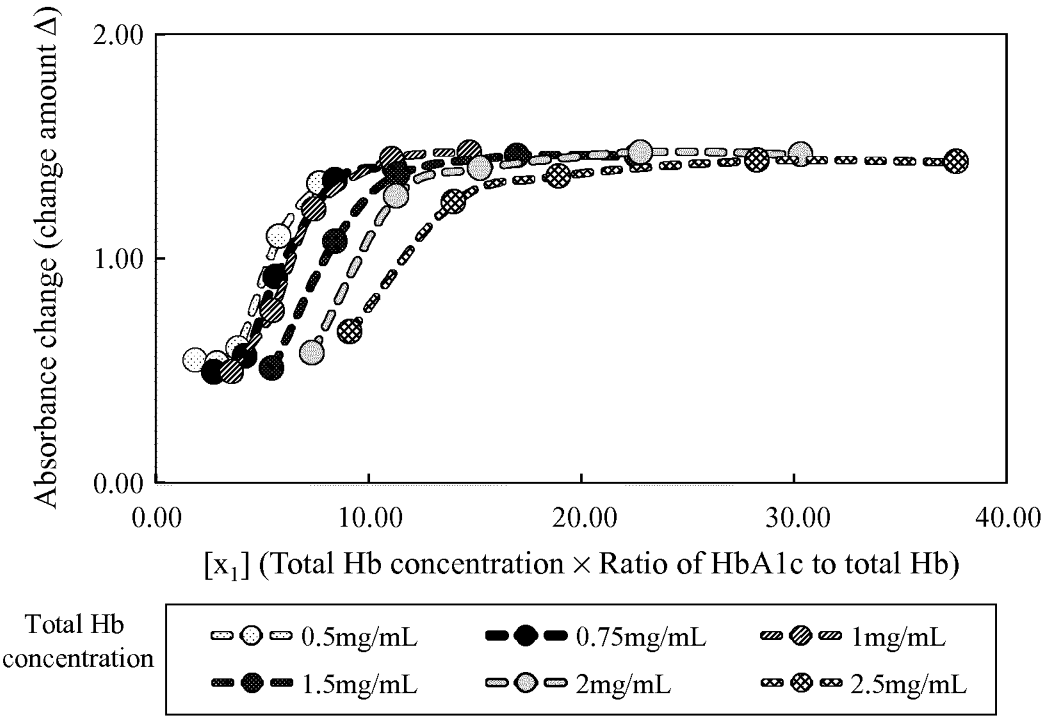
Absorbance change (change amount Δ)
2.00
1.00
0.00
0.00
10.00
20.00
30.00
40.00
[x1] (Total Hb concentration × Ratio of HbA1c to total Hb)
Total Hb concentration
0.5mg/mL
0.75mg/mL
1mg/mL
1.5mg/mL
2mg/mL
2.5mg/mL

Fig. 7A

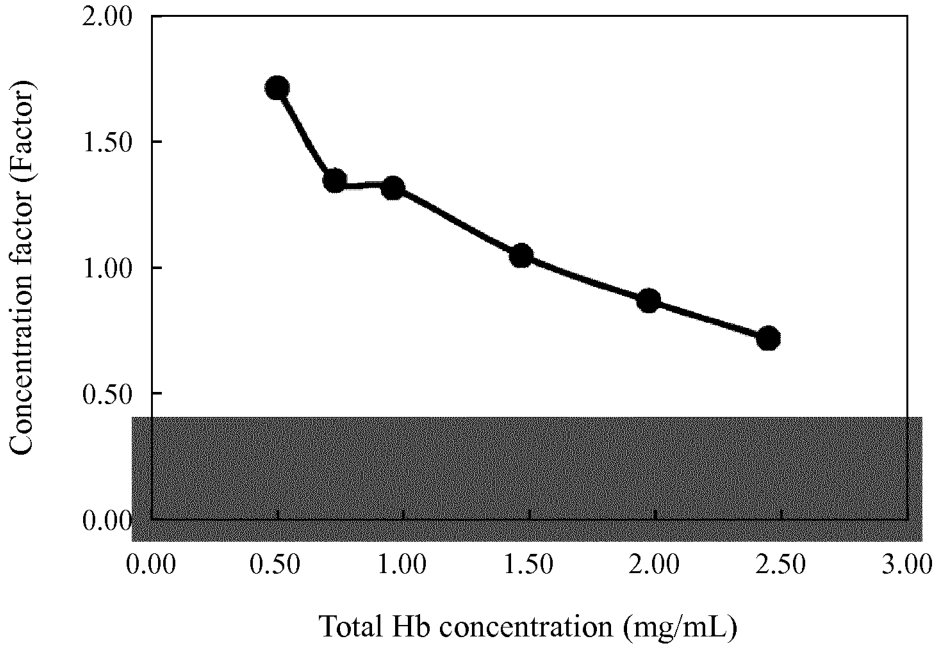
Concentration factor (Factor)
2.00
1.50
1.00
0.50
0.00
0.00
0.50
1.00
1.50
2.00
2.50
3.00
Total Hb concentration (mg/mL)

Fig. 7B

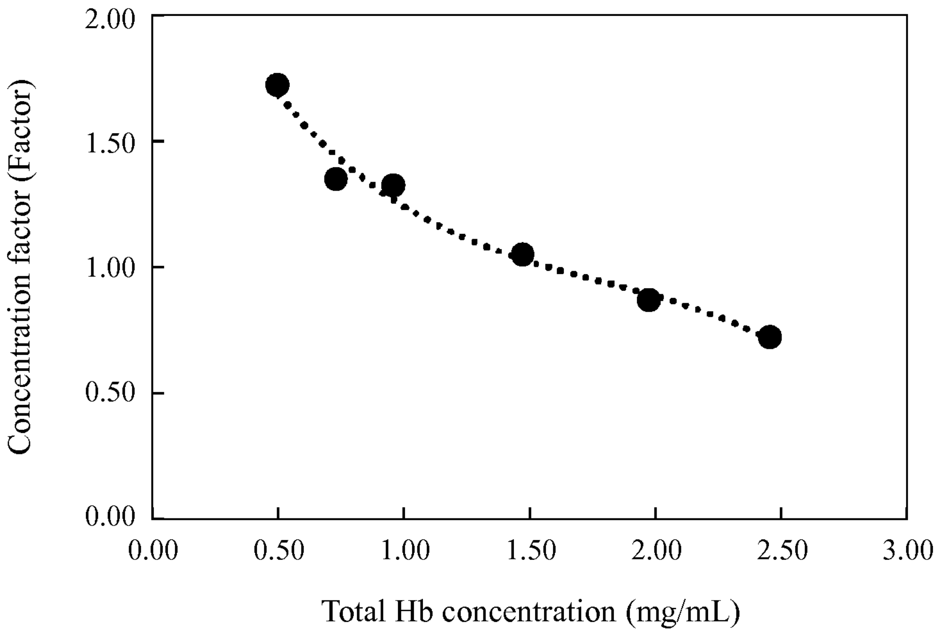
Concentration factor (Factor)
2.00
1.50
1.00
0.50
0.00
0.00
0.50
1.00
1.50
2.00
2.50
3.00
Total Hb concentration (mg/mL)

Fig. 8

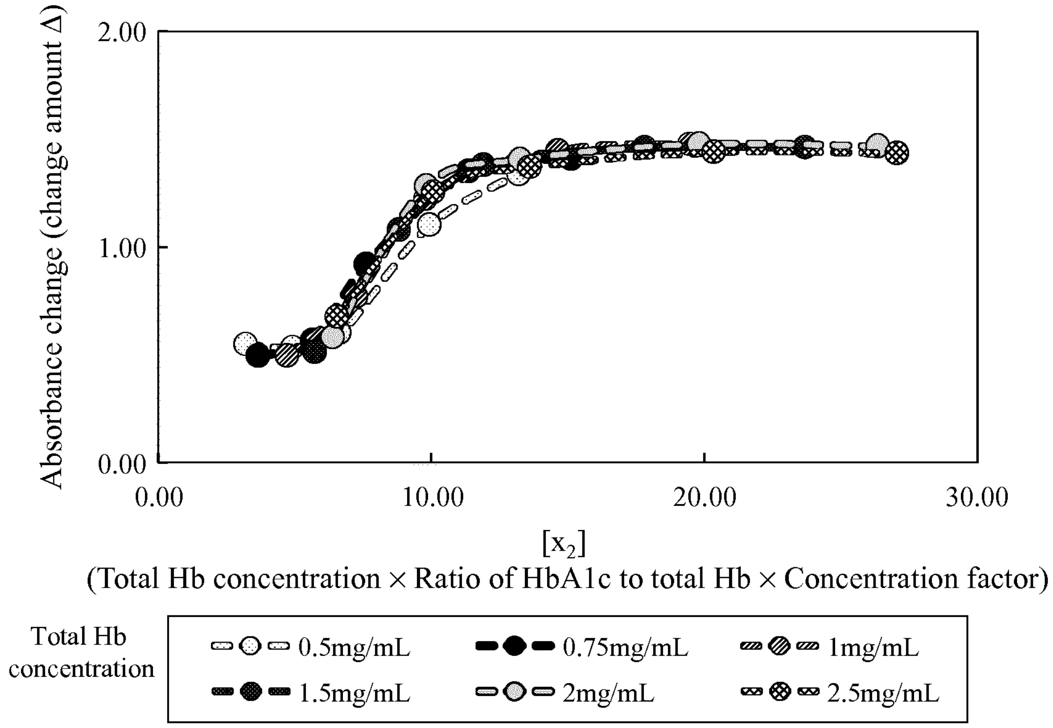
2.00
1.00
0.00
Absorbance change (change amount Δ)
0.00
10.00
20.00
30.00
[$x_2$]
(Total Hb concentration × Ratio of HbA1c to total Hb × Concentration factor)
Total Hb concentration
0.5mg/mL
0.75mg/mL
1mg/mL
1.5mg/mL
2mg/mL
2.5mg/mL

Fig. 9

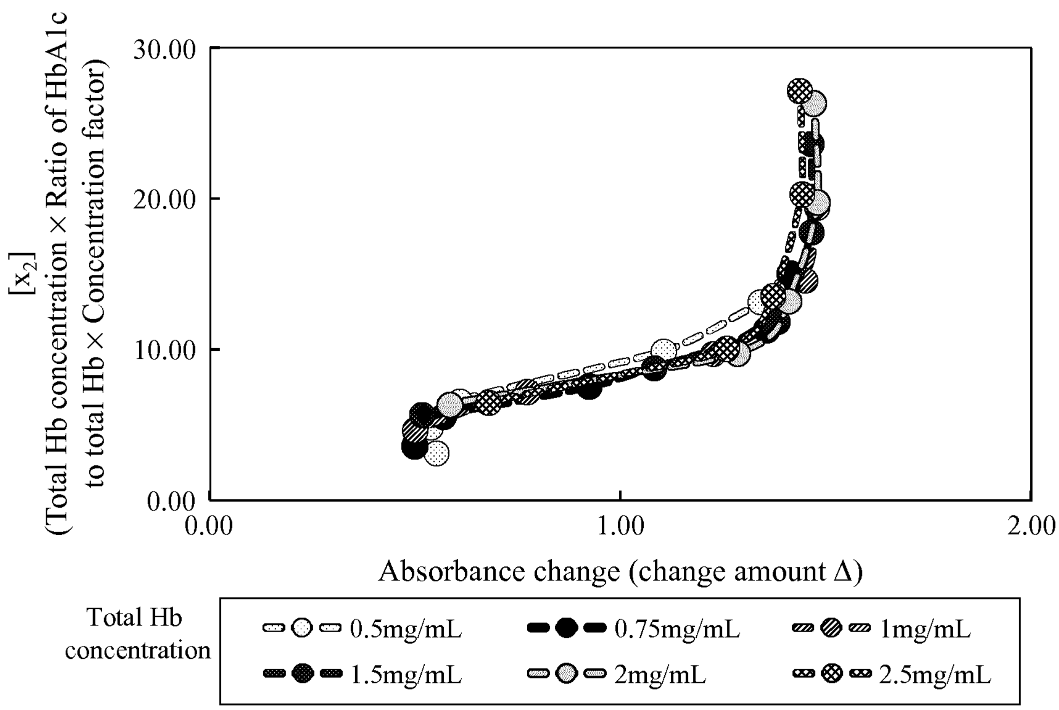
30.00
20.00
10.00
0.00
[$x_2$]
(Total Hb concentration × Ratio of HbA1c to total Hb × Concentration factor)
0.00
1.00
2.00
Absorbance change (change amount Δ)
Total Hb concentration
0.5mg/mL
0.75mg/mL
1mg/mL
1.5mg/mL
2mg/mL
2.5mg/mL

Fig. 10

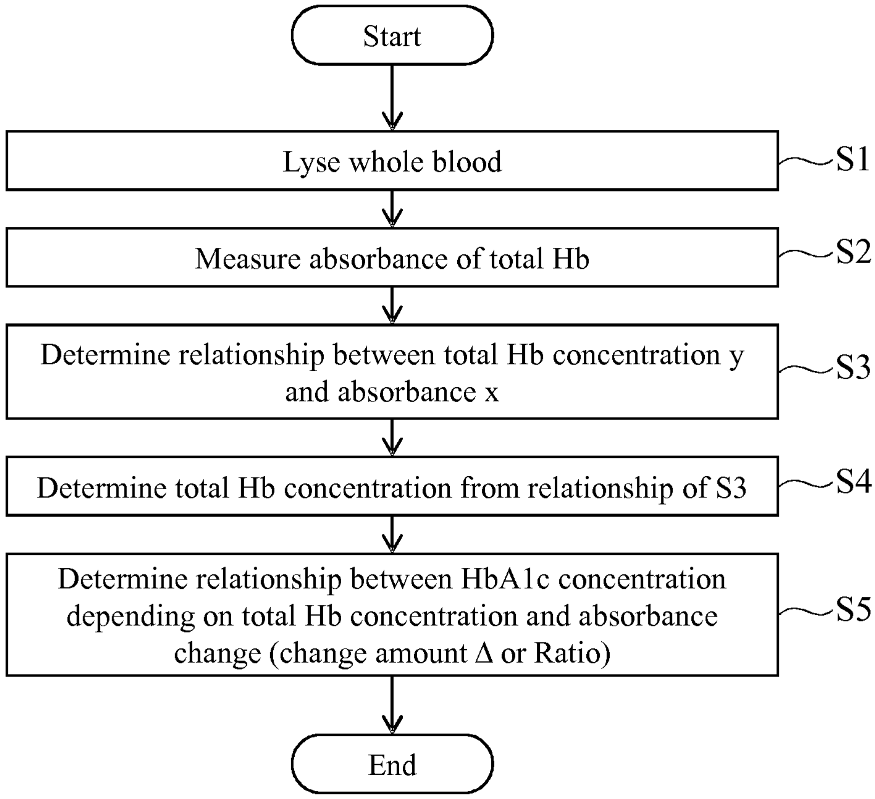


Fig. 11A

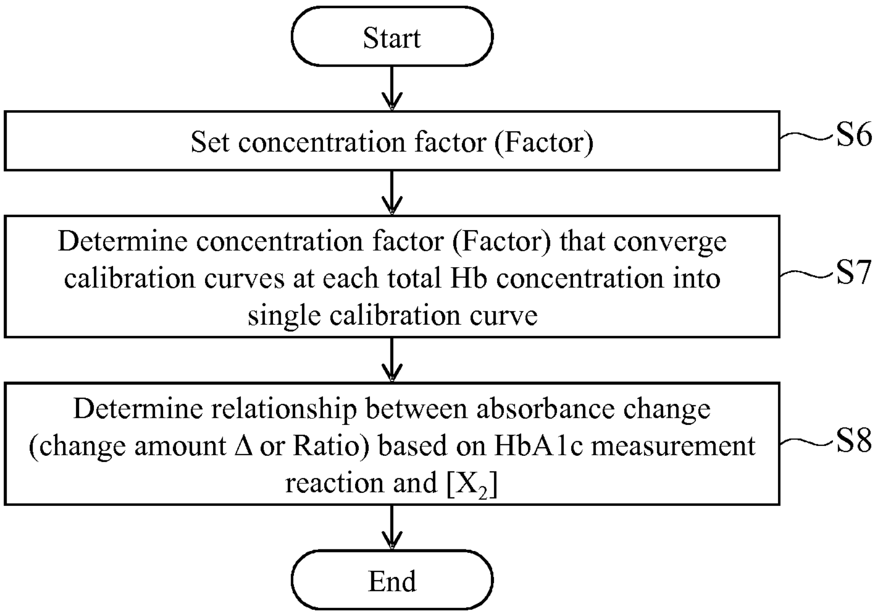

Fig. 11B

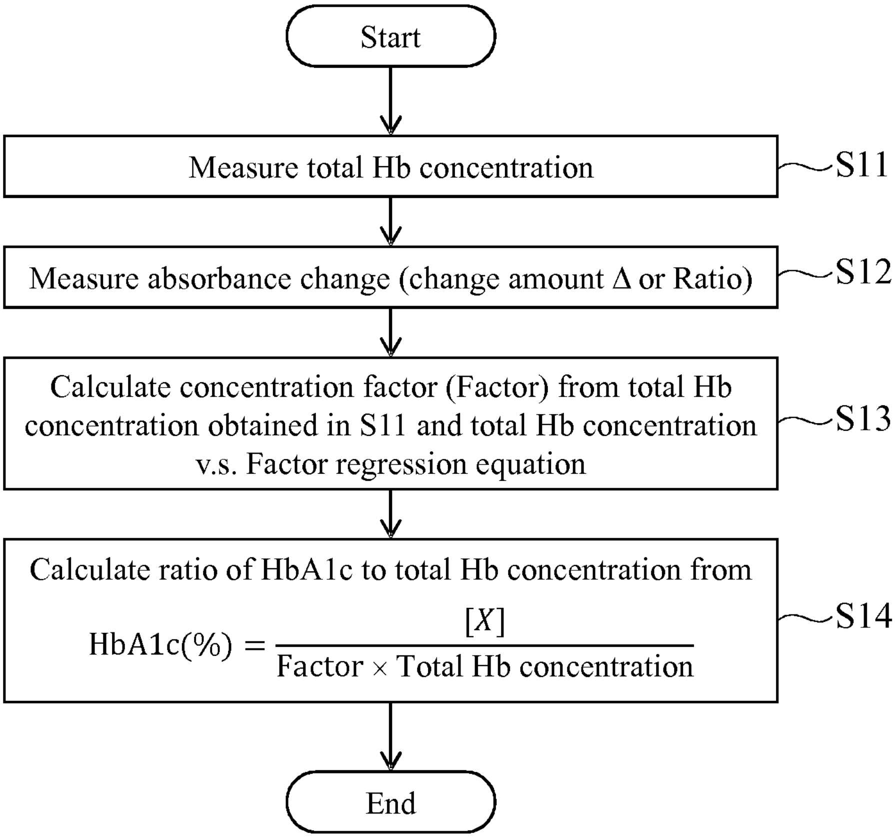
Start
Measure total Hb concentration
S11
Measure absorbance change (change amount Δ or Ratio)
S12
Calculate concentration factor (Factor) from total Hb concentration obtained in S11 and total Hb concentration v.s. Factor regression equation
S13
Calculate ratio of HbA1c to total Hb concentration from
$HbA1c(\%) = \frac{[X]}{Factor \times Total\ Hb\ concentration}$
S14
End

Fig. 12

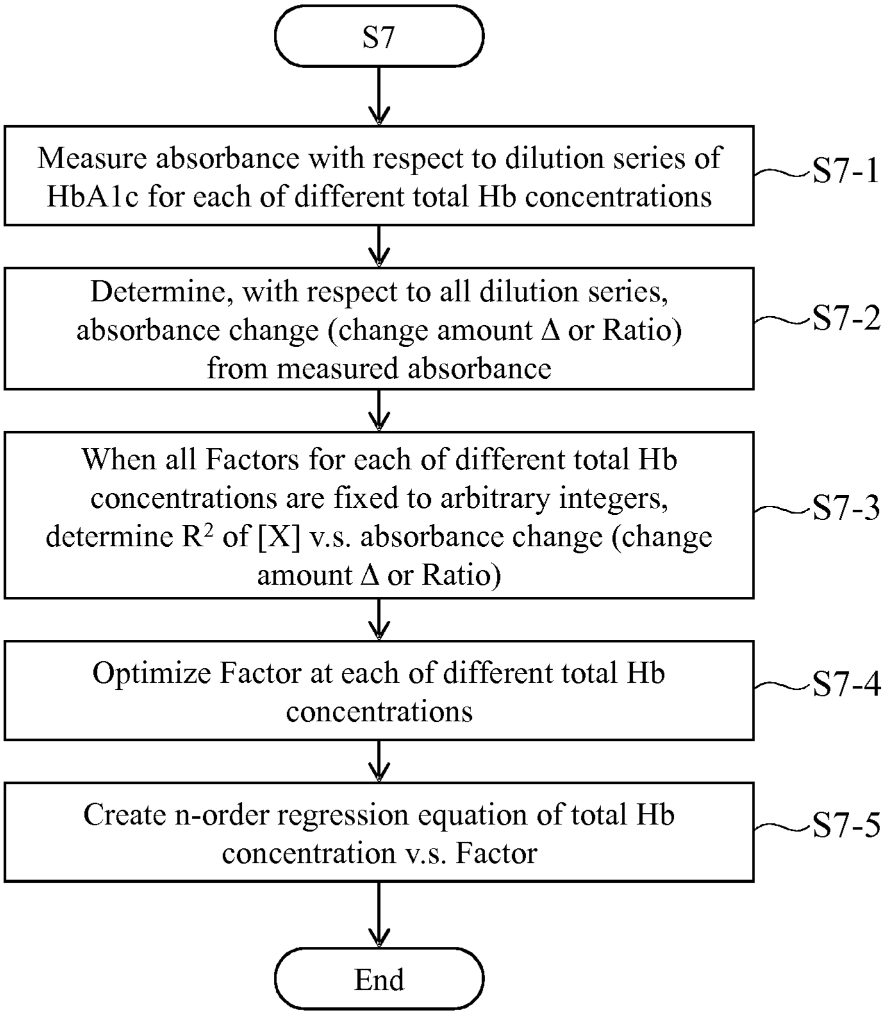
S7
Measure absorbance with respect to dilution series of HbA1c for each of different total Hb concentrations
S7-1
Determine, with respect to all dilution series, absorbance change (change amount Δ or Ratio) from measured absorbance
S7-2
When all Factors for each of different total Hb concentrations are fixed to arbitrary integers, determine $R^2$ of [X] v.s. absorbance change (change amount Δ or Ratio)
S7-3
Optimize Factor at each of different total Hb concentrations
S7-4
Create n-order regression equation of total Hb concentration v.s. Factor
S7-5
End

Fig. 13

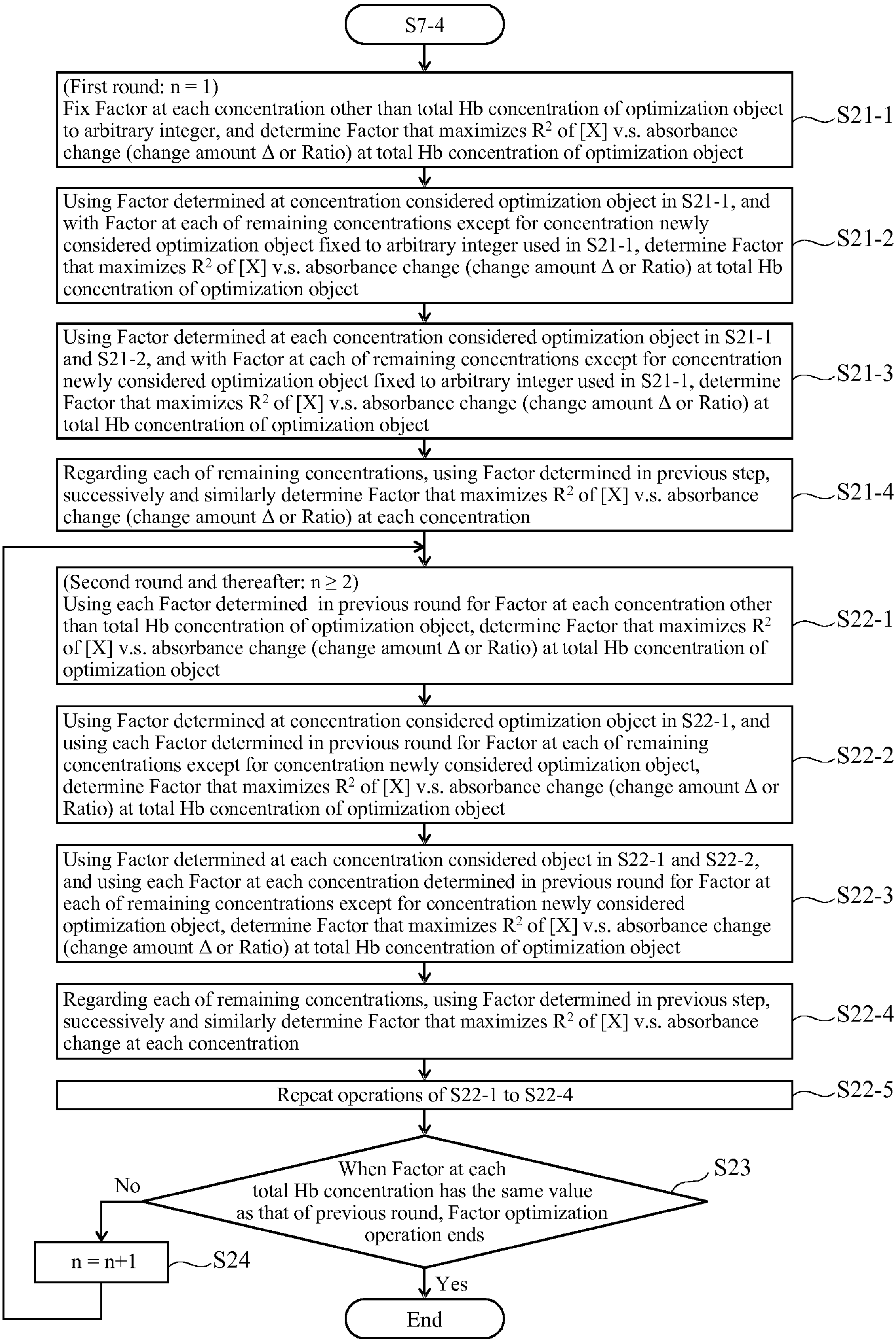
S7-4
(First round: n = 1)
Fix Factor at each concentration other than total Hb concentration of optimization object to arbitrary integer, and determine Factor that maximizes $R^2$ of [X] v.s. absorbance change (change amount Δ or Ratio) at total Hb concentration of optimization object
S21-1
Using Factor determined at concentration considered optimization object in S21-1, and with Factor at each of remaining concentrations except for concentration newly considered optimization object fixed to arbitrary integer used in S21-1, determine Factor that maximizes $R^2$ of [X] v.s. absorbance change (change amount Δ or Ratio) at total Hb concentration of optimization object
S21-2
Using Factor determined at each concentration considered optimization object in S21-1 and S21-2, and with Factor at each of remaining concentrations except for concentration newly considered optimization object fixed to arbitrary integer used in S21-1, determine Factor that maximizes $R^2$ of [X] v.s. absorbance change (change amount Δ or Ratio) at total Hb concentration of optimization object
S21-3
Regarding each of remaining concentrations, using Factor determined in previous step, successively and similarly determine Factor that maximizes $R^2$ of [X] v.s. absorbance change (change amount Δ or Ratio) at each concentration
S21-4
(Second round and thereafter: n ≥ 2)
Using each Factor determined in previous round for Factor at each concentration other than total Hb concentration of optimization object, determine Factor that maximizes $R^2$ of [X] v.s. absorbance change (change amount Δ or Ratio) at total Hb concentration of optimization object
S22-1
Using Factor determined at concentration considered optimization object in S22-1, and using each Factor determined in previous round for Factor at each of remaining concentrations except for concentration newly considered optimization object, determine Factor that maximizes $R^2$ of [X] v.s. absorbance change (change amount Δ or Ratio) at total Hb concentration of optimization object
S22-2
Using Factor determined at each concentration considered object in S22-1 and S22-2, and using each Factor at each concentration determined in previous round for Factor at each of remaining concentrations except for concentration newly considered optimization object, determine Factor that maximizes $R^2$ of [X] v.s. absorbance change (change amount Δ or Ratio) at total Hb concentration of optimization object
S22-3
Regarding each of remaining concentrations, using Factor determined in previous step, successively and similarly determine Factor that maximizes $R^2$ of [X] v.s. absorbance change at each concentration
S22-4
Repeat operations of S22-1 to S22-4
S22-5
When Factor at each total Hb concentration has the same value as that of previous round, Factor optimization operation ends
S23
No
n = n+1
S24
Yes
End

Fig. 14

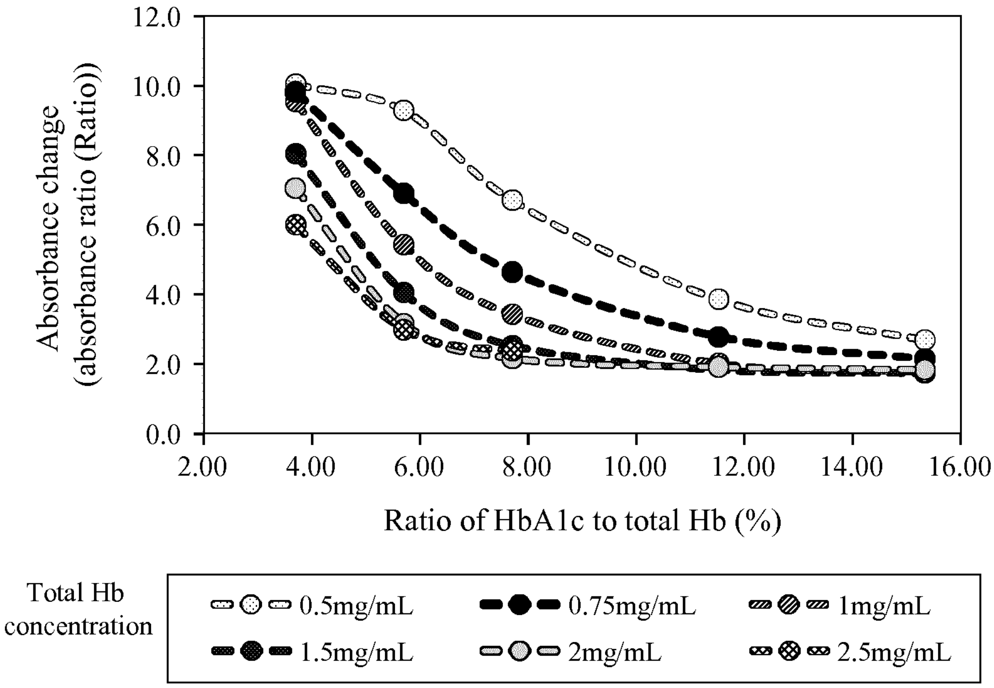
12.0
10.0
8.0
6.0
4.0
2.0
0.0
2.00
4.00
6.00
8.00
10.00
12.00
14.00
16.00
Absorbance change
(absorbance ratio (Ratio))
Ratio of HbA1c to total Hb (%)
Total Hb concentration
0.5mg/mL
0.75mg/mL
1mg/mL
1.5mg/mL
2mg/mL
2.5mg/mL

Fig. 15

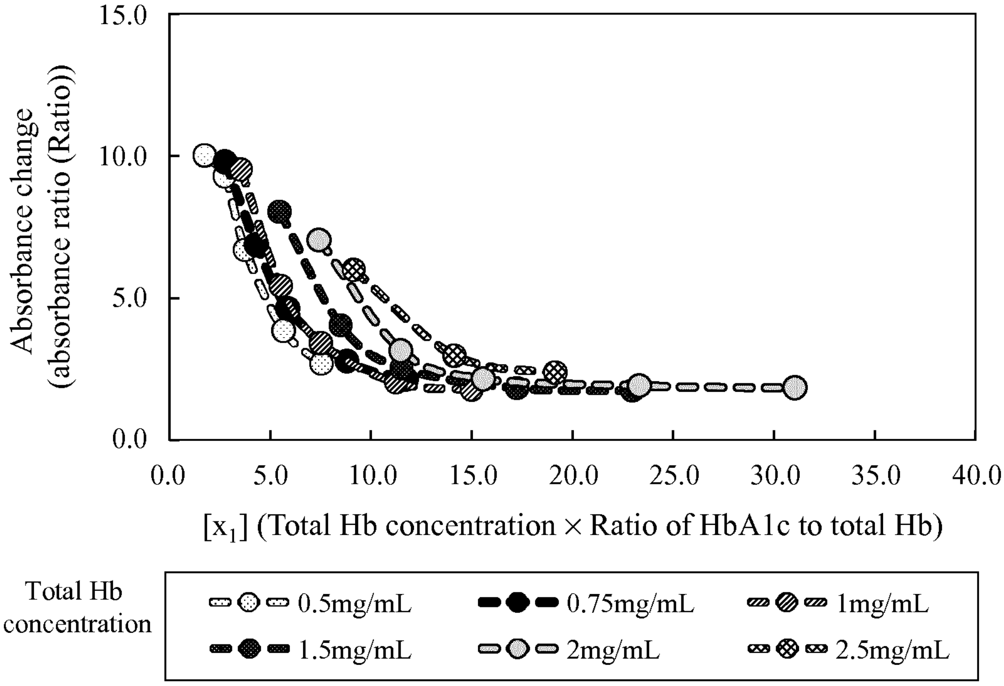
15.0
10.0
5.0
0.0
0.0
5.0
10.0
15.0
20.0
25.0
30.0
35.0
40.0
Absorbance change
(absorbance ratio (Ratio))
[x1] (Total Hb concentration × Ratio of HbA1c to total Hb)
Total Hb concentration
0.5mg/mL
0.75mg/mL
1mg/mL
1.5mg/mL
2mg/mL
2.5mg/mL

Fig. 16A

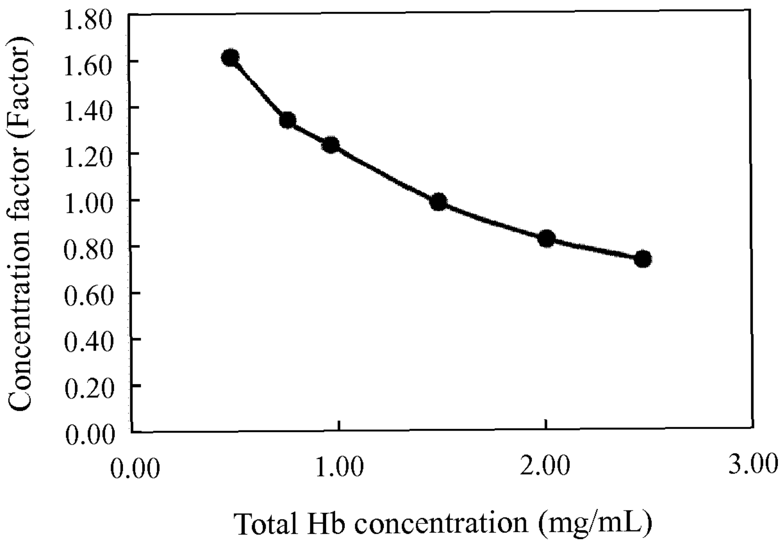


Fig. 16B

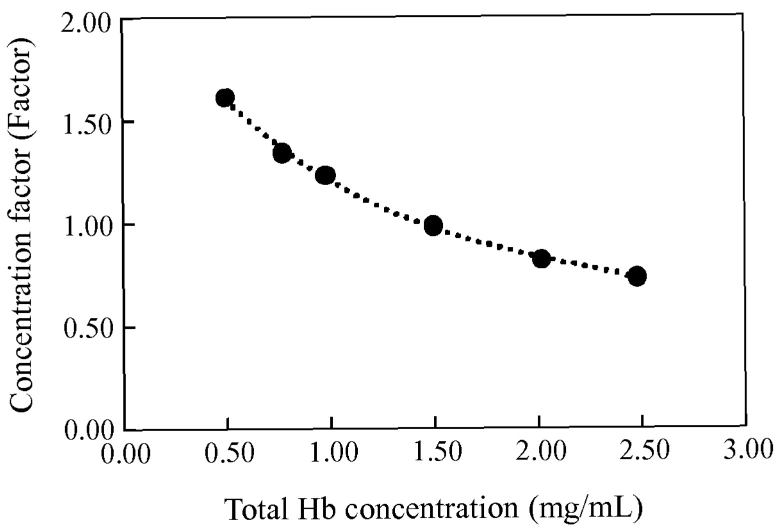

Fig. 17

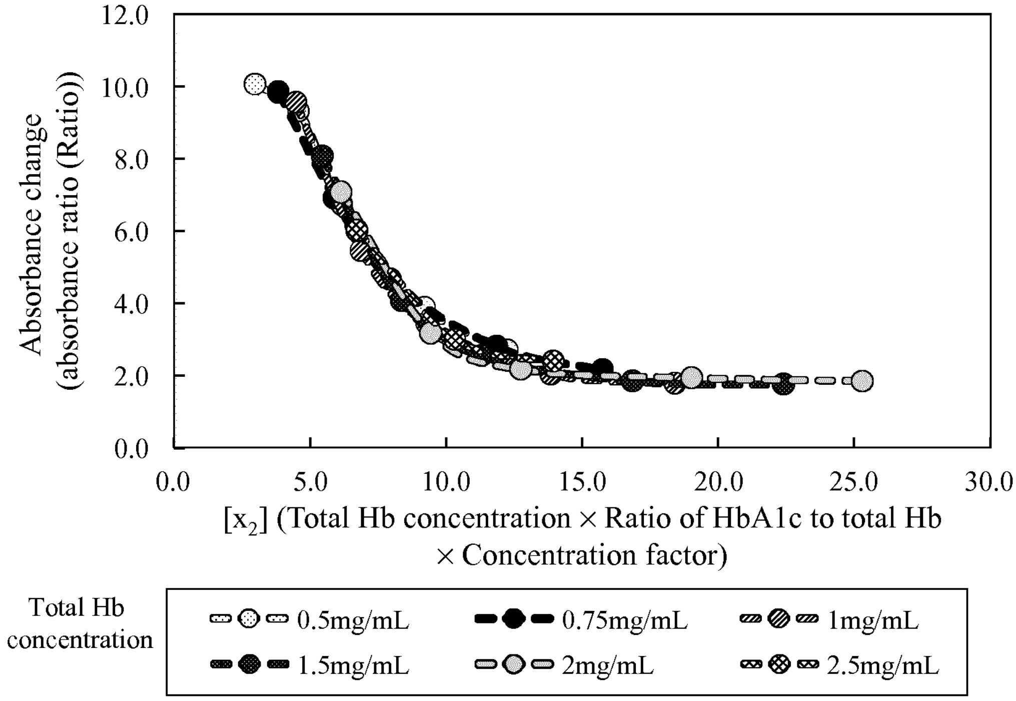
Absorbance change
(absorbance ratio (Ratio))
12.0
10.0
8.0
6.0
4.0
2.0
0.0
0.0
5.0
10.0
15.0
20.0
25.0
30.0
[x2] (Total Hb concentration × Ratio of HbA1c to total Hb
× Concentration factor)
Total Hb
concentration
0.5mg/mL
0.75mg/mL
1mg/mL
1.5mg/mL
2mg/mL
2.5mg/mL

Fig. 18

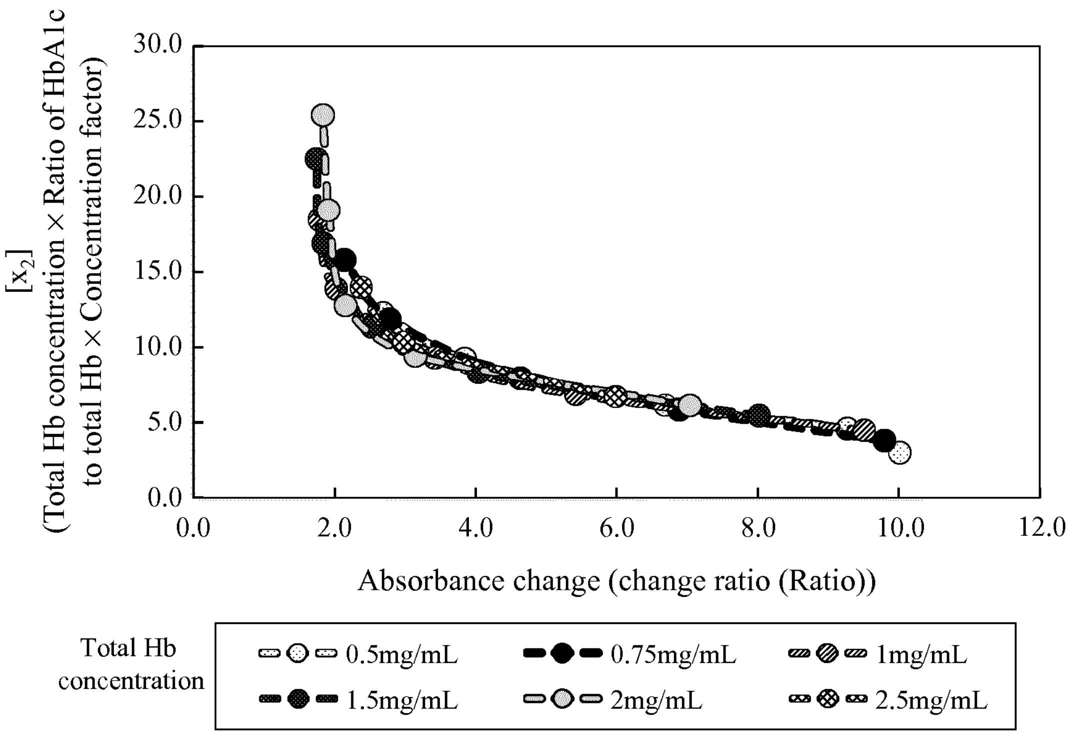
[x2]
(Total Hb concentration × Ratio of HbA1c
to total Hb × Concentration factor)
30.0
25.0
20.0
15.0
10.0
5.0
0.0
0.0
2.0
4.0
6.0
8.0
10.0
12.0
Absorbance change (change ratio (Ratio))
Total Hb
concentration
0.5mg/mL
0.75mg/mL
1mg/mL
1.5mg/mL
2mg/mL
2.5mg/mL

Fig. 19

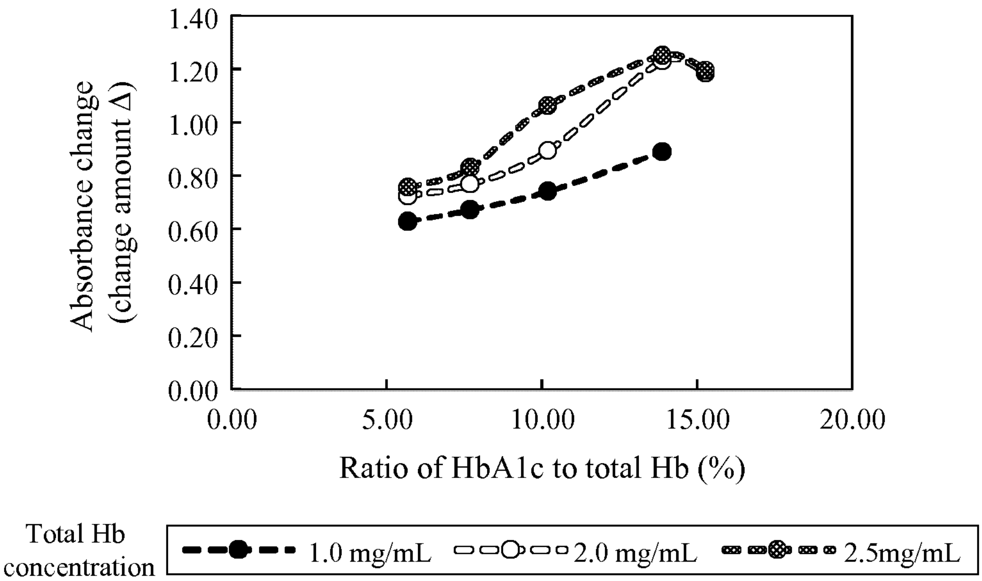
Absorbance change
(change amount Δ)
1.40
1.20
1.00
0.80
0.60
0.40
0.20
0.00
0.00
5.00
10.00
15.00
20.00
Ratio of HbA1c to total Hb (%)
Total Hb
concentration
1.0 mg/mL
2.0 mg/mL
2.5mg/mL

Fig. 20

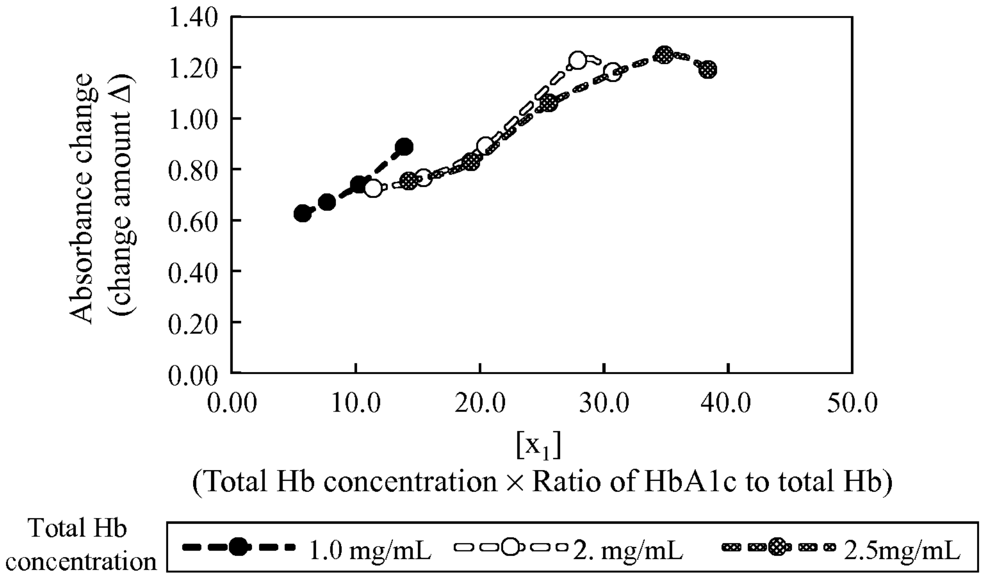
Absorbance change
(change amount Δ)
1.40
1.20
1.00
0.80
0.60
0.40
0.20
0.00
0.00
10.0
20.0
30.0
40.0
50.0
[x1]
(Total Hb concentration × Ratio of HbA1c to total Hb)
Total Hb
concentration
1.0 mg/mL
2. mg/mL
2.5mg/mL

Fig. 21A

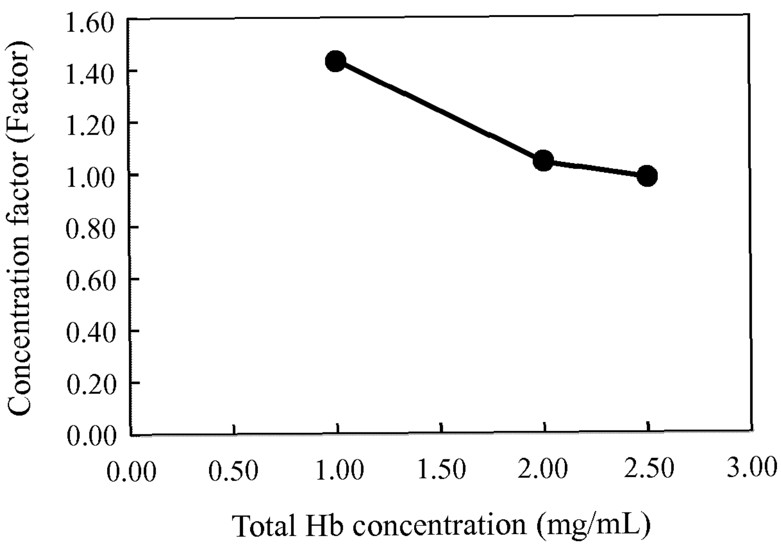


Fig. 21B

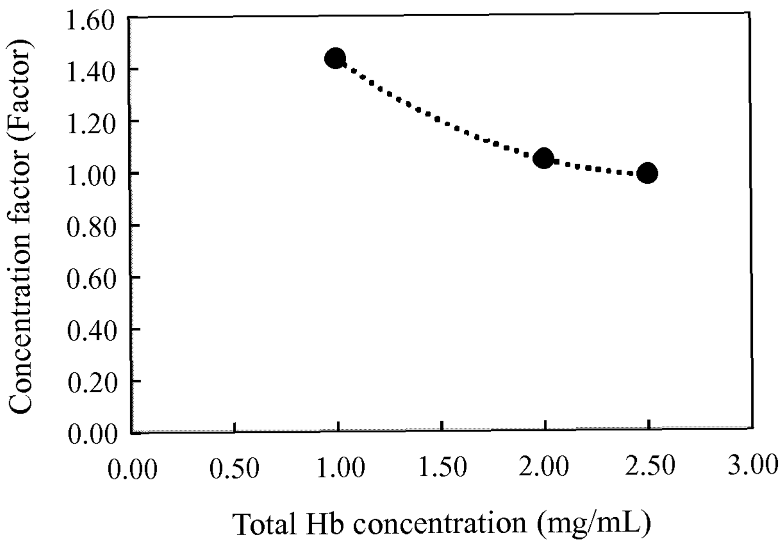

Fig. 22

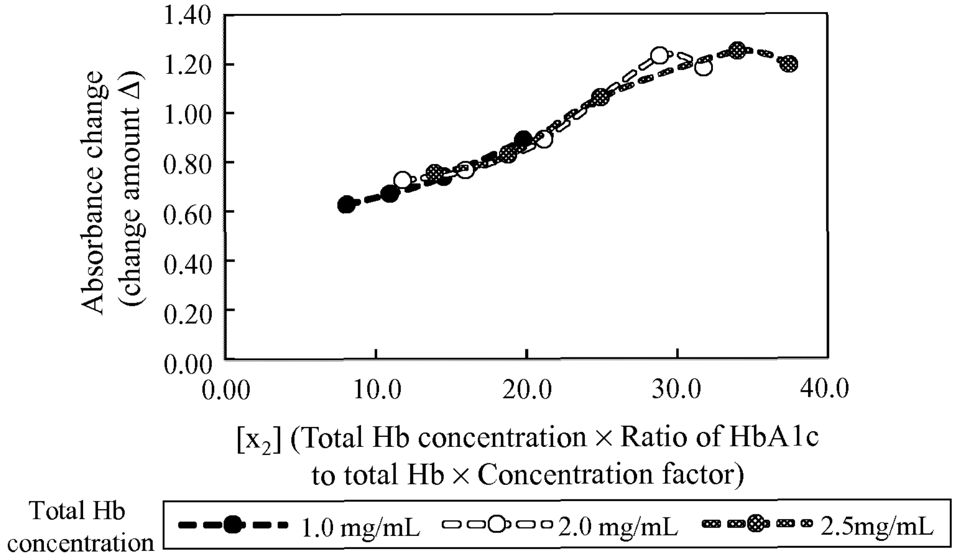
1.40
1.20
1.00
0.80
0.60
0.40
0.20
0.00
Absorbance change
(change amount Δ)
0.00
10.0
20.0
30.0
40.0
[$x_2$] (Total Hb concentration × Ratio of HbA1c
to total Hb × Concentration factor)
Total Hb
concentration
1.0 mg/mL
2.0 mg/mL
2.5mg/mL

Fig. 23

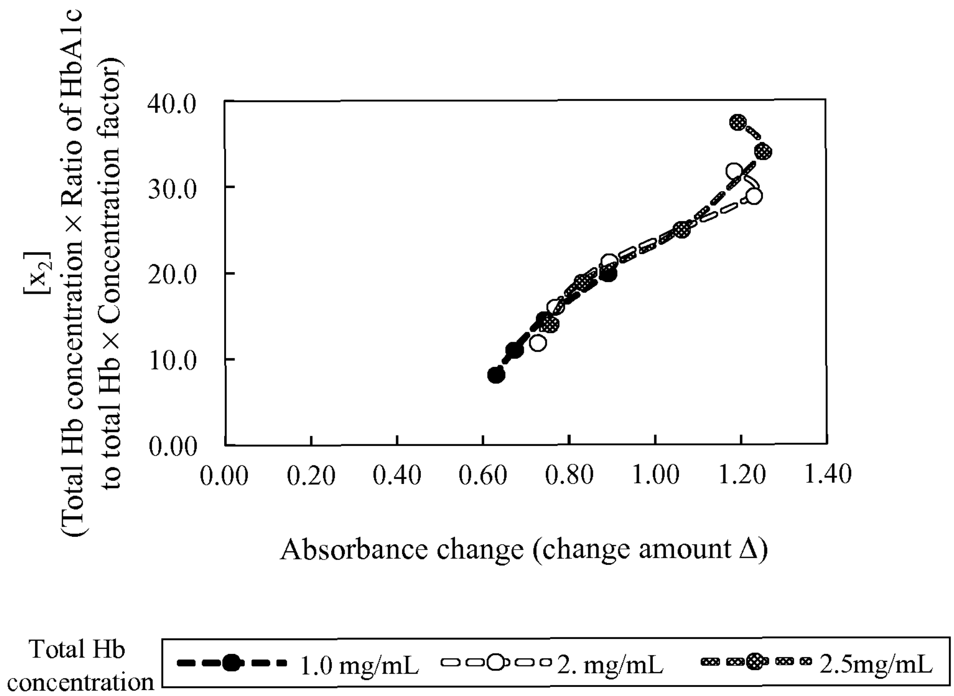
[$x_2$]
(Total Hb concentration × Ratio of HbA1c
to total Hb × Concentration factor)
40.0
30.0
20.0
10.0
0.00
0.00
0.20
0.40
0.60
0.80
1.00
1.20
1.40
Absorbance change (change amount Δ)
Total Hb
concentration
1.0 mg/mL
2. mg/mL
2.5mg/mL

## INTERNATIONAL SEARCH REPORT

International application No.
PCT/JP2017/017734

A. CLASSIFICATION OF SUBJECT MATTER
*G01N33/543*(2006.01)i

According to International Patent Classification (IPC) or to both national classification and IPC

B. FIELDS SEARCHED

Minimum documentation searched (classification system followed by classification symbols)
G01N33/543

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | WO 2002/006519 A1 (Arkray, Inc.), 24 January 2002 (24.01.2002), entire text; all drawings; particularly, claims; examples & JP 2007-181466 A & JP 3971702 B2 & US 2003/0162242 A1 entire text; all drawings; particularly, claims; examples & EP 1304385 A1 & EP 2336351 A2 & EP 2944699 A1 | 1-10 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July 2017 (28.07.17) | 08 August 2017 (08.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No. PCT/JP2017/017734

C (Continuation). DOCUMENTS CONSIDERED TO BE RELEVANT

| Category* | Citation of document, with indication, where appropriate, of the relevant passages | Relevant to claim No. |
|---|---|---|
| A | WO 2012/173185 A1 (Kyowa Medex Co., Ltd.), 20 December 2012 (20.12.2012), entire text; all drawings; particularly, paragraphs [0117] to [0121] & JP 2017-23155 A & US 2015/0132786 A1 entire text; all drawings; particularly, paragraphs [0113] to [0115] & EP 2722396 A1 | 1-10 |
| A | WO 2011/126067 A1 (Toyobo Co., Ltd.), 13 October 2011 (13.10.2011), entire text; all drawings; particularly, examples & JP 2015-165827 A | 1-10 |
| A | JP 2005-261383 A (Asahi Kasei Pharma Corp.), 29 September 2005 (29.09.2005), entire text; particularly, claims; paragraphs [0009], [0010] (Family: none) | 1-10 |
| A | JP 2009-531700 A (Quotient Diagnostics Ltd.), 03 September 2009 (03.09.2009), entire text; all drawings; particularly, claims; paragraphs [0027] to [0040] & JP 5118126 B2 & US 2009/0176309 A1 entire text; all drawings; particularly, claims; paragraphs [0045] to [0073] & GB 2436681 A & WO 2007/113590 A2 & EP 2005164 A2 | 1-10 |
| A | JP 2007-003410 A (Sekisui Chemical Co., Ltd.), 11 January 2007 (11.01.2007), entire text; all drawings (Family: none) | 1-10 |
| A | JP 2001-204495 A (Asahi Kasei Corp.), 31 July 2001 (31.07.2001), entire text (Family: none) | 1-10 |
| A | JP 01-237453 A (Hitachi, Ltd.), 21 September 1989 (21.09.1989), entire text; all drawings & US 5083283 A & DE 3908831 A | 1-10 |
| A | WO 2016/071887 A1 (ALIFAX S.R.L.), 12 May 2016 (12.05.2016), entire text; particularly, claims (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2596322 B
- JP 3550614 B
- JP 5465008 B
- JP 2016096681 A

### Non-patent literature cited in the description

- *Clin Chem.,* 2005, vol. 51, A246
- *Journal of Bioscience and Bioengineering,* 2014, vol. 92 (2), 62-68